(19) Europäisches Patentamt / European Patent Office / Office européen des brevets

(11) **EP 3 944 844 B1**

(12) **EUROPEAN PATENT SPECIFICATION**

(45) Date of publication and mention
of the grant of the patent:
**23.08.2023 Bulletin 2023/34**

(21) Application number: **20188701.5**

(22) Date of filing: **30.07.2020**

(51) International Patent Classification (IPC):
*A61F 13/532* *(2006.01)*   *A61F 13/56* *(2006.01)*
*A61F 13/531* *(2006.01)*   *A61F 13/49* *(2006.01)*

(52) Cooperative Patent Classification (CPC):
**A61F 13/49007; A61F 13/531; A61F 13/5323;**
**A61F 13/5638;** A61F 2013/5315

(54) **TAPED ABSORBENT ARTICLES WITH FRONT AND CROTCH CHANNELS**

ABSORBIERENDE ARTIKEL MIT KLEBESTREIFEN MIT VORDER- UND SCHRITTKANÄLEN

ARTICLES ABSORBANTS EN BANDE AYANT DES CANAUX À L'AVANT ET À L'ENTREJAMBE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**02.02.2022 Bulletin 2022/05**

(73) Proprietor: **The Procter & Gamble Company**
**Cincinnati, OH 45202 (US)**

(72) Inventors:
• **BIANCHI, Ernesto Gabriel**
**65824 Schwalbach am Taunus (DE)**
• **KREUZER, Carsten Heinrich**
**65824 Schwalbach am Taunus (DE)**

(74) Representative: **P&G Patent Germany**
**Procter & Gamble Service GmbH**
**Sulzbacher Straße 40**
**65824 Schwalbach am Taunus (DE)**

(56) References cited:
**WO-A1-2012/170778**    **US-A1- 2014 163 506**
**US-A1- 2014 371 701**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

FIELD OF THE INVENTION

The invention relates to personal hygiene absorbent articles to absorb body exudate such as urine and feces.

BACKGROUND OF THE INVENTION

Absorbent articles for personal hygiene such as diapers are designed to absorb and contain body exudates, in particular large quantity of urine. Diapers can be typically divided in two categories: taped articles or pant-like articles. The taped articles comprise a pair of fastening tapes at the back of the article that can be fastened to a landing zone material at the front of the article to form a closed article. The article is typically applied on the wearer by first laying the article down and open with its inner side facing up. The wearer is then laid with the buttocks towards the middle of this inner side of the article. The front half and the lateral sides of the article are then folded over the legs and front crotch of the wearer, and the article is closed around the legs and waist of the wearer by fastening the tapes to the landing zone material. Pant-type articles on the other hand have pre-sealed side seams and are put on like an underwear by sliding the pre-formed leg holes along the legs of the wearers up to the wearer's crotch.

Absorbent articles such as diapers typically comprise a liquid permeable topsheet on the wearer-facing side, a liquid impermeable backsheet on the garment-facing side and an absorbent core for absorbing the fluid exudates between these two layers. The absorbent core typically comprises a layer of absorbent material sandwiched between a core upper substrate layer and a lower substrate layer. The lower substrate layer and the upper substrate layer are typically sealed along the longitudinal edges of the core, and may be either open or sealed at their front and back edges. The upper substrate layer and lower substrate layer are typically a low basis weight nonwoven or a paper tissue, and may be referred together as the core wrap.

The absorbent material typically comprises superabsorbent polymers (SAP), as is known in the art. The SAP is typically in the form of small particles, which may be mixed with cellulose fibers. The SAP typically represents from 40% to 70% by the weight of the absorbent material, the rest being cellulose fibers. More recently, so called pulp-less or airfelt-free absorbent cores, wherein the absorbent material does not comprise cellulose fibers, have been marketed. In these airfelt-free cores, the SAP particles may be enclosed in pockets, see for example US5,433,715 (Tanzer et al.), WO2012/052172 (Van Malderen), or are immobilized by a fibrous network of adhesive fibers, see for example US2008/312617 (Hundorf et al).

Absorbent cores comprising channels in the absorbent layer that are substantially free of absorbent material have been proposed. These channels may help directing the fluid over a longer area of the absorbent core in a more efficient way than by diffusion, or help the core conforming to a desired shape during use. Various channel constructions have been proposed in the art. Channels formed without bonds between the upper substrate layer and lower substrate layer quickly disappear as the absorbent material swells and fills the channels or even earlier through the movements of the wearer. Bonded channels where the upper substrate layer of the core is bonded to the lower substrate layer of the core through the channels have also been proposed. Bonded permanent channels can remain in place even after the absorbent core has absorbed a substantial amount of fluid. The upper substrate layer and the lower substrate layer may be bonded in the channels using known bonding techniques such as adhesive bonding, mechanical bonding, thermo-bonding, or ultra-sonic bonding. Examples of such bonded channels are disclosed for example in WO2012/170778A1, WO2012/170779A1 (both Rosati et al.) and WO2014/93129 (Roe et al.). Advantages of such bonded channels are improved fit, less core sagging and better fluid distribution during use. Channels with temporary bonds that gradually open during use to free up more space for the swelling SAP have also been suggested, for example as disclosed in WO2014/200794 (Bianchi et al.). Such temporary channels can be advantageous to release more volume for the absorbent material to swell in as the absorbent core is loaded, while keeping the benefits of permanent channels at lower load. WO2019/083767A1 (Bianchi) discloses absorbent articles comprising channels at different positions in the absorbent core and having different bond strength to allow for a gradual, controlled opening of the channels.

The present invention is directed to absorbent articles comprising crotch channels and front channels in their absorbent cores. It was found that front channels extending relatively close to the front edge of the article can provide the benefit of keeping the diaper close to the front body of the wearer and preventing the front edge of the article flipping or folding when the tapes are fastened in non-optimal manner on the landing zone. It was also found that such front channels can reduce the core protrusion at the front of the article when the absorbent material swells, and maintain adequate longitudinal stiffness. However, in this configuration with front channels placed close to the front edge of the absorbent article, the front channels are at least partially superposed with the landing zone where the fastening tapes are fastened during use. It was found that a three-dimensional peak and valley profile materializes following the front channel shapes as the core swells, with the landing zone material deforming in a similar way. When the absorbent material swells to relatively high load, this causes stress on the landing zone which may consequently accidently release the fastening tapes. The present invention addresses these issues.

## SUMMARY OF THE INVENTION

The invention is in a first aspect directed to a taped absorbent article, and in a second aspect to a process for making such an article, as indicated in the claims. The absorbent core of the article comprises an absorbent material layer disposed between an upper substrate layer and a lower substrate layer. The absorbent material layer comprises at least one crotch channel disposed predominantly in the crotch region of the article, and at least one front channel disposed predominantly in the front region of the article, the front channel being at least partially superposed with the landing zone.

While the upper substrate layer and the lower substrate layer are bonded to each other through the crotch channel(s), according to the invention, the front channel(s) is (are) less strongly bonded, or even not substantially bonded, so that the absorbent material can more easily cause the upper substrate layer to delaminate from the lower substrate layer in the front channel(s) when the absorbent material swells after absorbing a fluid.

The present invention significantly reduces the stress caused by swelling absorbent material under the landing zone where the front channels are present, relieving the above mentioned problem of accidental unfastening of the tapes from the landing zone. The crotch channels, on the other hand, have a crotch channel bond strength which is higher than the channel strength of the front channels. The crotch channels can thus perform their functions such as fluid distribution in the longitudinal direction of the absorbent layer over a larger absorbed load range.

Further advantageous aspects of the present invention are disclosed in the following description and claims. The crotch channels and the front channels may in particular be each respectively provided as a pair of channels disposed symmetrically relative to the longitudinal axis of the article. The front channels may be separated from the crotch channels by a separating zone comprising absorbent material. The front and crotch channels optionally do not reach any of the edges of the absorbent material layer, and are thus fully encompassed within the absorbent material layer. The absorbent material may be a mixture of cellulose fibers and SAP particles, or alternatively the absorbent material may substantially consist of SAP particles without cellulose fibers. This and various other aspects of the invention are described in the following description and attached claims.

## BRIEF DESCRIPTION OF THE DRAWINGS

Fig. 1 is a top view of the wearer-facing side of an exemplary taped diaper which has been pulled flat, with some layers partially removed;
Fig. 2 shows a partial exploded view of the taped diaper of Fig. 1;
Fig. 3 shows a schematic transversal cross-section of the diaper of Fig. 1 in the region of the crotch channels;
Fig. 4a shows a schematic transversal cross-section of the diaper of Fig. 1 in the region of the front channels, wherein the front channels are bonded;
Fig. 4b shows an alternative schematic transversal cross-section of the diaper of Fig. 1 in the region of front channels, wherein the front channels are unbonded;
Fig. 5 is a top view of the exemplary absorbent core of the previous Figures shown in isolation and the upper substrate layer of the core partially removed;
Fig. 6 is a top view of an alternative core having a shaped absorbent layer;
Fig. 7 is a top view of an alternative core having straight channels;
Fig. 8 is a top view of an alternative core having crotch channels forming a X shape;
Figs. 9-12 illustrate how to conduct the Static Peel Force Time test further described herein.

For ease of discussion, the absorbent cores and articles of the invention will be discussed with reference to these Figures and the numerals referred therein, however these are not intended to limit the scope of the claims unless specifically indicated.

As used herein, the terms "comprise(s)" and "comprising" are open-ended; each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essentially of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "preferably", "advantageously", "in particular", "optionally" and the likes also qualify features which are not intended to limit the scope of the claims unless specifically indicated to do so.

## DETAILED DESCRIPTION OF THE INVENTION

### General description of an absorbent article 20

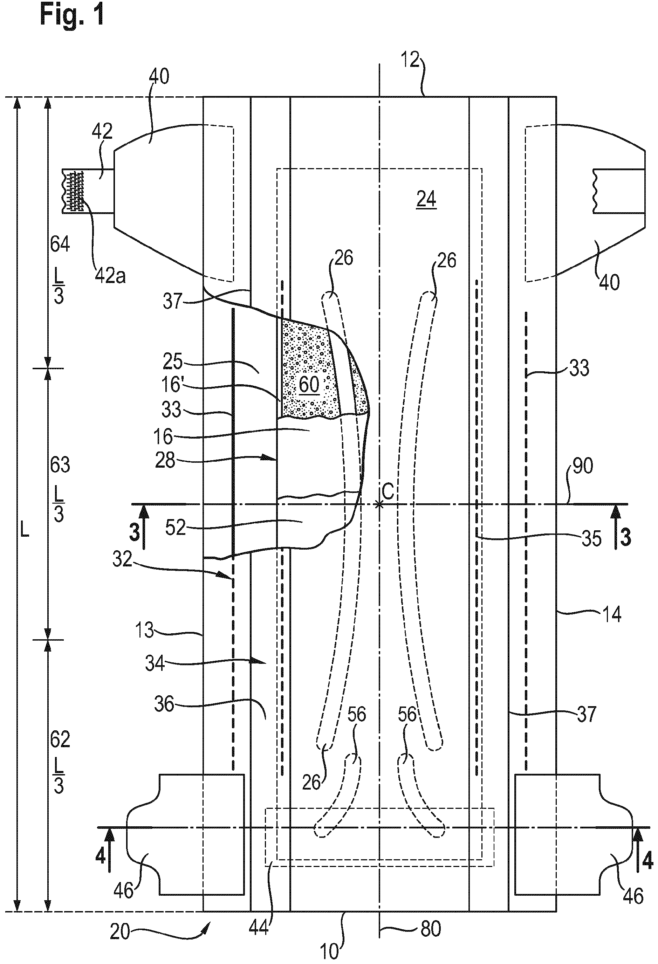

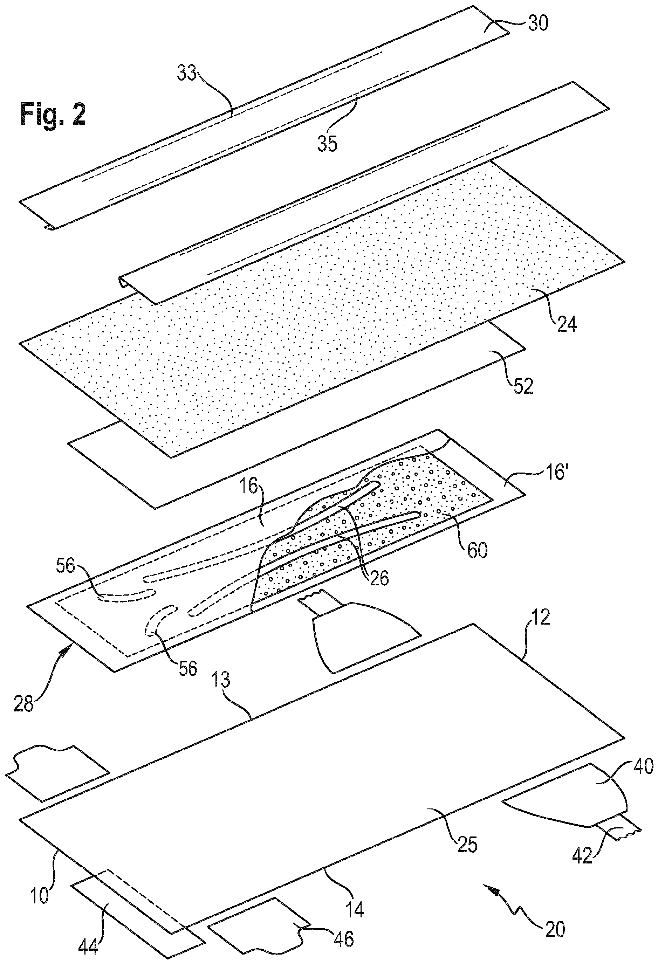

An exemplary absorbent article according to the invention in the form of a baby taped diaper 20 is represented in Figs. 1-4. Fig. 1 is a top plan view of the wearer-facing side of an exemplary diaper in a flat-out state, with portions of the structure being cut-away to more clearly show the construction of the diaper. Fig. 2 is an exploded view showing the different layers of the diaper of Fig. 1.

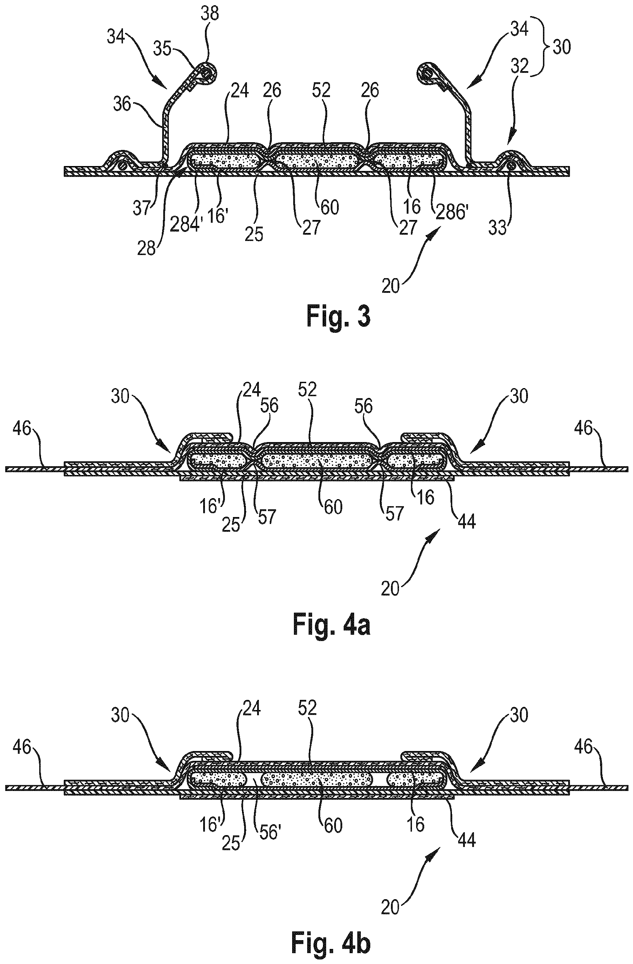

Figs. 3-4 are transversal cross-sectional view of the diaper 20 taken along lines 3-3, and 4-4 of Fig. 1 respectively. This diaper 20 is of course shown for illustration purpose only and is not limiting unless specifically indicated otherwise. In the following description the term "diaper" and "absorbent article" are used interchangeably.

As illustrated in Fig. 1, the absorbent article 20 comprises a front edge 10, a back edge 12, and two longitudinally-extending side (lateral) edges 13, 14. The front edge 10 is the edge of the article which is intended to be placed towards the front of the user when worn, and the back edge 12 is the opposite edge. The absorbent article is notionally divided by a longitudinal axis 80 extending along a longitudinal direction from the middle of the front edge 10 to the middle of the back edge 12 of the article and dividing the article in two substantially symmetrical halves relative to this axis, when viewing the article from the wearer-facing side in a flat out configuration, as exemplarily shown in Fig. 1. If some parts of the article are under tension due to elasticized components, the article may be typically flattened using clamps along the periphery of the article and/or a sticky surface, so that the article can be pulled taut so as to be substantially flat. Unless otherwise indicated, dimensions and areas disclosed herein apply to the absorbent article and absorbent core in this flat-out configuration.

The article has a length L as measured along the longitudinal axis 80 from the middle of the front edge 12 to the middle of the back edge 10. The absorbent article can also be notionally divided by a transversal axis 90. The transversal axis 90 is perpendicular to the longitudinal axis 80, which it crosses at half the length L. The intersection of the longitudinal axis 80 and the transversal axis 90 is defined herein as the centerpoint C of the article. The article can be further notionally divided in three regions having equal length of a third of L along the longitudinal axis: a front region 62 extending from the front edge 10 towards the crotch region for a third of L, a crotch region 63 in the middle third of the diaper, and a back region 64 extending from the crotch region to the back edge 12 of the article for the remaining third of L. All three regions are of equal length measured on the longitudinal axis, when the article is in such a flat state. The front region, crotch region, back region and longitudinal and transversal axis are defined herein notionally, that is they are typically not materialized in the real diapers, but are useful to describe the positions of various components of the invention relative to each other and the diaper.

The absorbent article 20 comprises a liquid-permeable topsheet 24, a liquid-impermeable backsheet 25 and an absorbent core 28 between the topsheet and the backsheet. The absorbent core comprises an absorbent material 60 sandwiched between an upper substrate layer 16 and a lower substrate layer 16'. The upper substrate layer and the lower substrate layer are together referred as core wrap. The absorbent material layer 60 has an outline as seen from the plane formed by the longitudinal and transversal axis which may be rectangular or shaped. The absorbent material layer 60 comprises at least one crotch channel 26 and at least one front channel 56. The crotch channel 26 is predominantly disposed in the crotch region of the article (it may extend into the front region and/or back region of the article) and the front channel 56 is predominantly disposed in the front region of the article. The crotch channel 56 comprises a bond 27 between the upper substrate layer and lower substrate layer, while the front channel has no bond or only a weaker 57 bond than the crotch channel bond. The front and crotch channels and their respective bonds are discussed further below in more details. The front and crotch channels respectively are preferably each disposed as a pair of channels symmetrically disposed relative to the longitudinal axis. The channels forming a pair may or may not be connected to each other along the longitudinal axis, the pair forming for example a U, V or X shape. As used herein, the term "a channel" or "at least one channel" means "one or more channel, in particular at least a pair of channels" unless specifically indicated otherwise.

The articles of the invention are taped articles, which comprise, as exemplarily illustrated in the diaper of Fig. 1, a pair of fastening tapes 42 provided on the back region of the article and a landing zone 44 on the front region of the article. The landing zone is typically a piece of discrete material, typically a nonwoven, that it attached to the external surface of the backsheet. The landing zone may also be integral with the nonwoven outer cover of the backsheet if such a layer is present, however this may increase the overall price of the backsheet compared to a discrete landing zone.

The wearer-facing side of the article is principally formed by the topsheet 24. A lotion (not represented) may be present, typically in longitudinally-extending stripes, directly on the topsheet. Other typical diaper components are represented in the Figures, such as elasticized gasketing cuffs 32 (also called outer cuffs), upstanding barrier leg cuffs 34 (also called inner cuffs, which are also typically elasticized). For clarity of the drawings, only one elastic strand 33, 35 is shown for each type of cuff, but typically each cuff may typically comprise from 1 to 4 elastic strands.

The absorbent article may further comprise one or more acquisition and/or distribution layers 52 between the topsheet and the absorbent core. An acquisition layer 52 may be disposed directly underneath the topsheet, with a distribution layer (not represented in the Figures) optionally present between the acquisition layer 52 and the absorbent core 28. Any acquisition or distribution layer may optionally also comprise channels (not represented) which are free of acquisition or distribution material. The different components of the articles are typically attached to adjacent components by any known means such as adhesive bonding, for example the topsheet may be attached to an acquisition layer, and the acquisition layer to the upper substrate layer of the absorbent core by spiral adhesive or slot coating as is known in the art. These attachments are not shown in the drawings for simplicity.

These and further components will be discussed in more details further below. The absorbent article may also comprise other typical components, which are not represented in the Figures, such as a back elastic waist feature, a front elastic waist feature, transverse barrier cuffs, a wetness indicator between the backsheet and the absorbent core that changes color when contacted with urine, etc...

### Fastening system 42, 44

The absorbent article comprises a pair of fastening tapes 42 on the back region of the article that can be fastened to a landing zone 44 on the front region of the article to form a closed article having a waist opening and two leg opening. This fastening system provides lateral tensions about the circumference of the absorbent article to hold the absorbent article around the wearer's waist. The fastening tapes 42 may be attached to stretchable back ears 40 disposed on each side of the back region of the article. The back ears 40 may be attached to the chassis of the article, as is known in the art, for example as disclosed in WO2006/138,725A2 (Lam et al.).

The fastening system may be according to any typical constructions known in the art. The fastening system typically comprisea interlocking fasteners, as known in hook-and-loop systems. The fastening tapes 42 typically comprise the "hooks" parts 42a, with the "loops" being part of the landing zone material, which typically comprise a nonwoven material with suitably shaped fibers. Other known fastening systems comprising interlocking fasteners are tabs & slots, buckles, buttons, snaps, and/or hermaphroditic fastening components. Any known fastening means are generally acceptable. The fastening system is preferably re-fastenable so the user can re-adjust the fastening of the tapes. The fasteners are typically releasably attached to the landing zone.

The landing zone 44 is provided on the front waist region of the article, with the fastening tape 42 engaging with the landing zone when the article is put on the wearer. While the landing zone is typically a discrete single material, it is not excluded that the landing zone comprise for example two separate portions disposed on each of the longitudinal axis and separated by a gap to engage respectively the left and right fastening tape.

Some exemplary fastening systems are disclosed in US3,848,594, US4,662,875, US4,846,815, US4,894,060, US4,946,527, US5,151,092 and US5,221,274 (Buell). An exemplary interlocking fastening system is disclosed in US 6,432,098. The fastening system may also provide a means for holding the article in a disposal configuration as disclosed in US4,963,140 (Robertson et al.)

### General description of an absorbent core 28

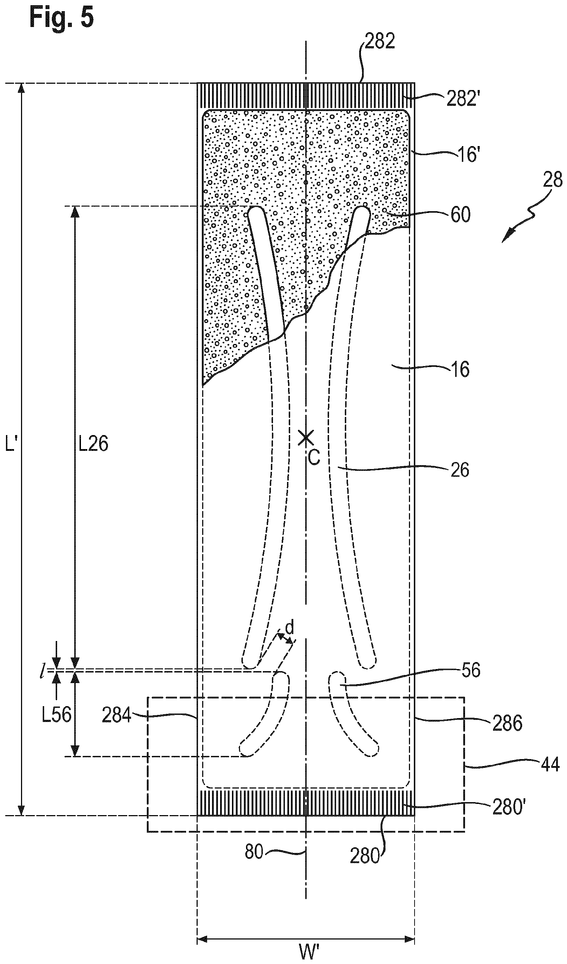

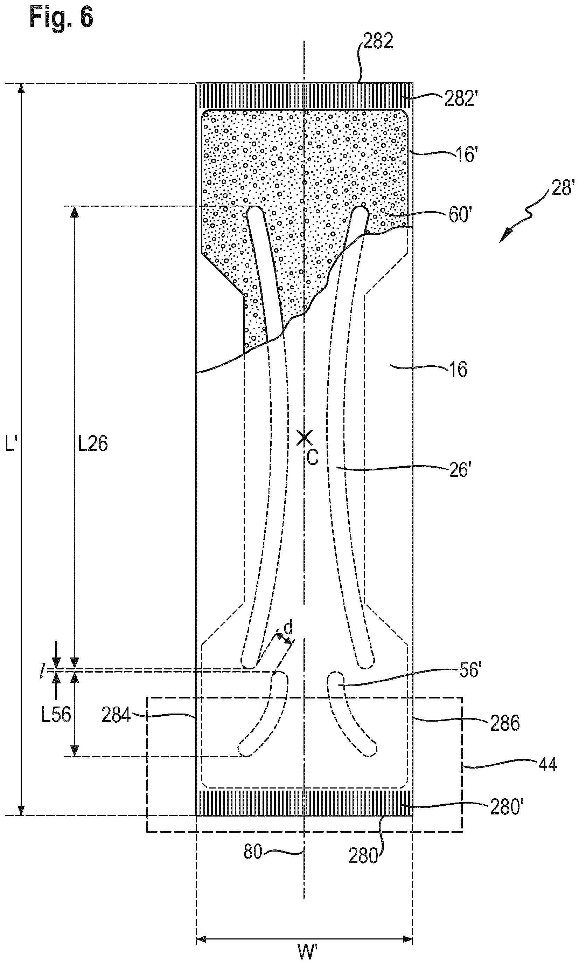

A first exemplary absorbent core 28 according to the invention is illustrated on Fig. 5 in isolation of the rest of the article 20 that was shown in Fig. 1. The position of the landing zone 44 is indicated in dotted line on Fig. 5, as well as in the other Figs. 6-8. As used herein, the term "absorbent core" or "core" refers to a component of an absorbent article which comprises an absorbent material layer 60 sandwiched between an upper substrate layer 16 and a lower substrate layer 16', referred together as core wrap. As used herein, the term "absorbent core" does not include the topsheet, the backsheet or a distribution/acquisition layer. The absorbent core has typically the most absorbent capacity of all the components of the absorbent article, and comprises all or at least the majority of superabsorbent polymer (SAP) in the article. The core typically thus consists essentially of, or consists of, the core wrap, the absorbent material and optionally adhesives. The terms "glue" and "adhesive" are used interchangeably. The absorbent material may consist of a blend of SAP particles and cellulose fibers, but the invention is also applicable to other absorbent material for example consisting to 100% of SAP particles and/or free of cellulose fibers. The terms "absorbent core" and "core" are herein used interchangeably.

The absorbent core may be typically thin and conformable, so that it can be laid on a flat surface as well as a curved surface, for example the curved surface of a drum during its making process. Fig. 5 shows a top view of the absorbent core of the article of Figs. 1-4. The upper substrate layer 16 forms the top side of the core, and the lower substrate layer 16' forms the bottom side of the absorbent core. The upper substrate layer and lower substrate layer may be formed by two separate substrates, but it is also possible to have a single substrate forming both top and lower substrate layers of the core wrap. The absorbent core typically comprises a front edge 280, a back edge 282 and two longitudinally-extending side edges 284, 286 extending between the front edge and the back edge. The front edge of the core 280 is the edge placed or intended to be placed towards the front edge 10 of the absorbent article. Typically the absorbent material may be advantageously distributed in somewhat higher amount towards the crotch region and front region than towards the back region as more absorbency is typically required towards the front half of the article. Typically the front and back edges 280, 282 are shorter than the longitudinally-extending side edges 284, 286. The upper substrate layer 16 of the core wrap is optionally more hydrophilic than the lower substrate layer 16'.

The absorbent material may also be profiled in cross-direction. In absorbent cores comprising a pair of crotch channels, the basis weight of the absorbent material may be profiled such that the basis weight of the absorbent material between the crotch channels is different than the basis of absorbent material between each channel and the corresponding longitudinal edge of the core. The basis weight may be higher or lower, or be both higher and lower depending on the longitudinal position considered, as for example taught WO2017/189150 A1 (Bianchi et al.). As indicated in this application, for absorbent cores comprising a pair of crotch channels, the central absorbent zone comprising absorbent material and disposed between the first and the second crotch channels, and the first and second lateral absorbent zones disposed respectively outwardly on each side of the crotch channels may be characterized in that the basis weight of the absorbent material in the central absorbent zone is higher than the basis weight of the absorbent material in each of the lateral absorbent zones for at least a first transversal section of the core having a first length in the longitudinal direction of at least 10 mm; and the basis weight of the absorbent material in the central absorbent zone is lower than the basis weight of the absorbent material in each of the lateral absorbent zones for at least a second transversal section of the core having a second length in the longitudinal direction of at least 10 mm.

On the other hand, it can advantageous that the basis weight of the absorbent material remains constant in cross-direction in the area where the front channels are present (for example for a pair of front channels, this means that the absorbent material's basis weight is the same between the front channels and between each channel and the corresponding absorbent layer's longitudinal edges). Keeping the basis weight constant in the area of the front channels can help the channels delaminating under the swelling pressure before creating a too rigid three dimensional structure that could impact tape functionality. However the basis weight of the absorbent material may also be profiled in the cross-machine direction in the area where the front channels are present.

The overall footprint of the absorbent core is defined by the core wrap and is typically generally rectangular with a core width W' in the transversal direction and a core length L' in the longitudinal direction as measured from edge to edge, including the region of the core wrap which does not enclose the absorbent material, in particular at the front end seal 280' and the back end seal 282' when present. The core wrap is typically longitudinally sealed and optionally transversally sealed at its back and front edges. If the core is not rectangular, the maximum dimension measured along the transversal direction and the longitudinal direction can be used to report the width and length of the core respectively. The width and length of the core may vary depending on the intended usage. For baby and infant diapers, the width W' may for example in the range from 40 mm to 200 mm and the length L' from 100 mm to 600 mm. Adult incontinence products may have higher maximum dimensions. The absorbent core may be symmetrical relative to the longitudinal axis 80. The longitudinal axis of the core typically overlaps with the longitudinal axis of the absorbent article when the core is incorporated in the article, so both longitudinal axis are designated by the same number 80 in the Figures.

The absorbent material may be any conventional absorbent material used in absorbent articles. The absorbent material may be a blend of cellulose fibers with superabsorbent polymer (SAP) particles, also called absorbent gelling materials (AGM), see for example US 5,151,092 (Buell). The absorbent material may in particular comprise, by weight, from 30% to 75% of SAP particles, in particular from 40% to 70% by weight of SAP particles, or from 45% to 65% by weight of SAP particles relative to the total weight of absorbent material. The rest of the absorbent material may typically be cellulose fibers. The absorbent material may thus comprise from 25% to 70% by weight of cellulose fibers. Synthetic fibers may also be comprised in the absorbent core but are not typically considered as absorbent material. The absorbent material may also comprise higher amount of SAP, up to 75%, 80% by weight of the absorbent material, or more, mixed with cellulose or other fibers. The absorbent cores may also consist essentially of SAP without cellulose fibers as absorbent material (so called "airfelt-free" cores) as known in the art. For example WO2008/155699 (Hundorf) discloses absorbent cores with a patterned layer of SAP immobilized by a net of fibrous thermoplastic adhesive material deposited over the layer of SAP.

Suitable SAP may be any water-insoluble, water-swellable polymers capable of absorbing large quantities of fluids, as is known in the art. The term "superabsorbent polymer" refers herein to absorbent materials, typically cross-linked polymeric materials, that can absorb at least 10 times their weight of an aqueous 0.9% saline solution as measured using the Centrifuge Retention Capacity (CRC) test (EDANA method WSP 241.2.R3 (12)). The SAP may in particular have a CRC value of more than 20 g/g, or more than 24 g/g, or of from 20 to 50 g/g, or from 20 to 40 g/g, or 24 to 30 g/g.

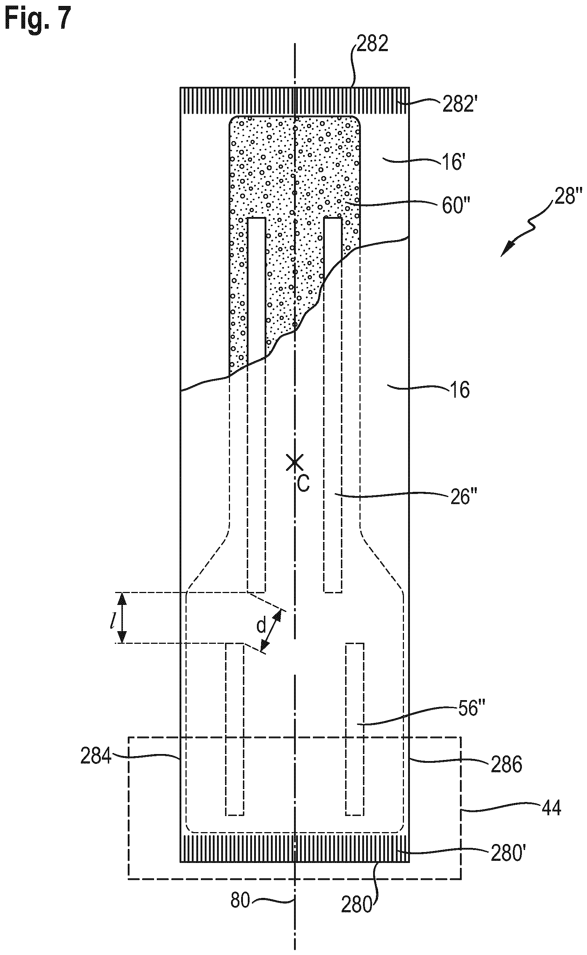

The absorbent material layer 60 may be generally rectangular as shown in Figure 5, but other shapes are possible. The absorbent layer may be shaped with the longitudinal edges 284, 286 of the cores having a tapered section in the crotch region relative to the front region and back region as illustrated in Fig. 6 for the shaped absorbent layer 60'. An alternatively shaped absorbent layer having a larger front area than the back area is shown in Fig. 7. Small size baby diapers may also comprise a notch on the front edge of the absorbent material layer to adapt to the presence of remains of the umbilical cord.

The absorbent core is not limited to a particular thickness (caliper). Typically, the thickness of the core (dry, i.e. before use) as measured at the centerpoint point (C) where the longitudinal and transversal axis of the article meet may range from 2.0 mm to 10.0 mm, in particular from 3.0 mm to 7.0 mm as measured at 2.07 kPa (0.30 psi) with a flat circular foot having a diameter of 17.0 mm ($\pm$ 0.2 mm).

The absorbent material may be deposited on any of the upper substrate layer or lower substrate layer using the layer as a deposition substrate, with the other layer being then applied or folded over the absorbent layer thus deposited. Alternatively, the absorbent material may be deposited as a first and second absorbent layers on the upper substrate layer 16 and lower substrate layer 16' respectively, with both absorbent layers being then brought in face to face contact and sandwiched together to form a unitary layer. This construction is for example used in certain air-felt free absorbent cores, such as disclosed in WO2008/155699 (Hundorf et al).

The absorbent cores of the present invention are not limited to a particular process for making them, and the cores of the invention may be more conventionally made by air-laying a mix of cellulose fibers and superabsorbent particles on a conventional air-laying drum fitted with raised portions matching the shapes of the desired channels so that that substantially no absorbent material is deposited in these areas. See for example WO2004/011,723, Venturino et al. for a modified drum having raised portions to create areas having different basis weight. The shape of the raised portions may be adapted to make any desired channel shapes.

**Crotch channels 26 and front channels 56**

The absorbent core 28 comprises at least a crotch channel 26 and at least a front channel 56 within the absorbent material layer 60. The channels are areas substantially free of absorbent material. "Substantially free" means that minute amount of absorbent material accidentally present due to involuntary contamination of the channels due to the high speed of the making process may be present. The singular form of the word channel is used herein to mean "one or more, in particular at least a pair of channels" unless specifically indicated otherwise, being it understood that a pair of channels will comprise one channel on one side of the longitudinal axis and the other channel symmetrically on the other side of the longitudinal axis.

The crotch channel 26 is predominantly present in the crotch region 63 of the article. The crotch channels can advantageously extend into the front region 62 and the back region 64 of the article, or least extend into the front region, or at least extend into the back region. These longitudinally extending channels are believed to provide for a better wet fit of the absorbent article as well as efficient utilization of the absorbent material. By "predominantly" present in the crotch region, it is meant that the length of the crotch channel is higher in the crotch region than in the front region or back region of the article. All length as indicated herein are measured as projected on the longitudinal axis 80 (or any other imaginary line parallel to the longitudinal axis). At least 40% of the crotch channel length may be in the crotch region, in particular at least 50% of the crotch channel length. For example the crotch channel may be configured such that at least 50% of its length is in the crotch region, and 25% or less in the back and front regions respectively.

The front channel 56 is predominantly present in the front region 62 of the article. By "predominantly" present, it is meant that at least 50% of the front channel length is in the front region of the article, in particular at least 60% and up to 100% (with 100% the front channel is entirely present in the front region and does not extend to the crotch region). The front channel 56 is at least partially superposed in vertical direction with the landing zone 44. According to the invention, the front channel extends relatively far towards the front edge of the article, so that the front channel is at least partially superposed (in the vertical direction) with the landing zone 44.

The cumulated length of all the channels of the absorbent core projected on the longitudinal axis is the sum of the length of all channels present in the core, but without double counting any overlap in case two channels are positioned at an overlapping position on the longitudinal axis. For example, on Fig. 5, the cumulated length of the channels is L56+L26 (the length of a crotch channel plus the length of a front channel). The cumulated projected length of all the channels in the core divided by the length of the core L' and expressed in percentage may advantageously range from 30% to 95%, preferably from 50% to 90%, in particular from 60% to 90%.

According to the present invention, the crotch channel 26 comprises a bond 27 between the upper substrate layer 16 and the lower substrate layer 16' of the core wrap, as illustrated in Fig. 3 for example. This bond 27 provides for structural integrity of the crotch channels in dry and wet state. Any known bonding techniques known in the art may be used to provide for this bond, in particular one selected from adhesive bonding, thermo bonding, mechanical bonding, ultrasonic bonding, or any combinations thereof. An inner core adhesive (not shown) may be for example applied at least in the areas of the channels on the upper substrate layer and/or the lower substrate layer of the core wrap, typically by slot glue application or any other means, followed by the application of pressure in the areas of the channels to provide a good adhesive bonding in these areas while the adhesive is still in its open state (for hotmelt adhesive). Exemplary patent disclosures of such adhesive bonding processes can be found for an airfelt or airfelt-free absorbent cores in WO2012/170,798A1 (Jackels et al.), EP2,905,000 (Jackels et al.) and EP2,905,001 (Armstrong-Ostle et al.).

Other bonding means such as thermo bonding, mechanical bonding, ultrasonic bonding can also be used as additional bonding or as an alternative bonding. For example, in the crotch channels an adhesive bonding may be reinforced by a thermo bonding , mechanical bonding, or ultra-sonic bonding. Such thermo, mechanical or ultrasonic bonding can be applied on the channel areas through the external sides of the core wrap, see for example WO95/11652 (Tanzer et al.) disclosing secondary bonding comprising a water-resistant adhesive or a thermo bond.

Typically the crotch channel bonds 27 may generally have the same outline and shape as the absorbent material free channel 26 in which they are contained, with the bonds being slightly smaller to allow for a tolerance margin (e.g. by a few mm) as some deviations from the optimal registration may happen during high speed process. It is expected that channel bonds 27 may be more efficiently made and stronger if they are provided in macroscopic areas with no absorbent material (except of course accidental contamination) compared to bonds provided in areas containing non-negligible amount of absorbent material. Typical channel width ranges from 2 mm to 20 mm, in particular from 4 mm to 12 mm.

The absorbent core further comprises at least one front channel 56 wherein the upper substrate layer 16 and lower substrate layer 16' of the core wrap are less strongly bonded than in the crotch channel(s). These two layers may also have no substantial bond through the front channels. Un-bonded channels can be obtained for example by not providing an adhesive, or if an adhesive is present by not purposively pressing the substrates together while the adhesive is still tacky, or by not providing any other bonding means in the area of the front channels, so that the top side and the bottom side of the core wrap in these areas will not bond to each other. This is for example illustrated in Figs. 4b for the front channels 56'.

The front channels with no or weaker bonds can provide for an initial quick fluid distribution in the front area of the core where they are present. However these front channels are not as permanent as the crotch channels, and once the absorbent layer absorbs fluid and swell, the upper and lower substrate layers in these front channels can delaminate due to the absorbent material swelling pressure, and thus can immediately or progressively release extra volume that can be filled up with the expanding absorbent material.

Non-permanent bonds can be provided by an inner core adhesive (also called auxiliary glue) which is typically present on the inner surface of the upper substrate layer and/or lower substrate layer forming the core wrap. Such inner core adhesive is typically used to partially immobilize the absorbent material within the core wrap. The inner core adhesive is typically applied broadly on at least one side of the core wrap by slot coating in the form of longitudinal stripes or spiral coating on the inner surface of a substrate as is known in the art. In some airfelt-free cores, the SAP particles are also immobilized using an additional fibrous network of glue. Thus in the cases where an adhesive is present within the core wrap, it may be difficult or complex to avoid applying any adhesive in the relatively narrow zones within the deposition area that correspond to the front channels. However, these adhesives will not provide channel bonds or only weak bonds if the adhesive is provided at relatively low basis weight and/or if no or little pressure is applied between the two sides of the core wrap in these regions during the making process of the core. An hotmelt adhesive will typically cool in a few seconds and loose its adhesive properties after this open time, so that if no or little pressure is applied in the channel areas directly after the application, an auxiliary glue or fibrous network hotmelt adhesive will not create a bond, or at least a much weaker one than if a strong pressure was applied after a few milliseconds following glue application for example. Alternatively or additionally, more of the same adhesive or a different adhesive (herein supplementary adhesive) may be applied in the area of the crotch channels but not in the front channels to provide stronger bonds in the crotch channels than in the front channels. Alternatively or additionally, additional bonding such as thermo-bonding or ultrasonic bonding may be used in addition to any of the structural adhesives indicated previously in both type of channels.

To sum up, the crotch channels are intentionally provided with relatively strong bonds between the core's upper layer and lower layer, for example by using a first bonding means such as an inner core adhesive and an additional bonding means such as: additional pressure application, a supplementary adhesive, a thermo-, ultrasonic- or mechanical bonding (such as embossing) specifically for these channels. The front channels on the other hand are provided with weaker bonds 57 as illustrated in Fig. 4a or without bonds as illustrated in Fig. 4b. If weaker bonds 57 are present in the front channels, these bonds may for example comprise the same first bonding means as for the crotch channels, but not additional bonding means of the crotch channel bonds. The front channel bonds 57 if they are present will thus more easily delaminate than the bonds 27 of the crotch channels. During use, the front channel bond 57 will thus delaminate more quickly than the crotch channel bond 27. The crotch channel bonds may even be substantially permanent, so that they do not typically release during a prolonged wear time of the article.

The strength of the bonds between the upper substrate layer and the lower substrate layer in a channel can be characterized by its Static Peel Force Time. In short, this parameter measures the time in minutes it takes for a 1-inch (2.54 cm) wide sample to delaminate when one side of the sample is hanging with a 150 g weight attached to it. The Static Peel Force Time of the crotch channels is thus higher than the Static Peel Force Time of the front channels. In particular, the Static Peel Force Time of the crotch channel may be higher than 50 minutes, or higher than 60 minutes or higher than 70 minutes, while the Static Peel Force Time of the front channel is less than 50 minutes, in particular less 40 minutes or less than 30 minutes. The crotch channel may have a Static Peel Force Time which is at least 20 minutes, or at least 30 minutes, or at least 40 minutes, higher than the Static Peel Force Time of the front channel.

The crotch channels and the front channels may be generally formed in the same manner, except for the bonding step for the crotch channels, which does not exist or is much weaker for the front channels. Herein, when the term "channels" is used without further indication, it is meant any or all of the crotch channels and/or front channels, unless it is clear from the context that a particular type of channels was meant.

It is advantageous in terms of side leakage prevention that all, or at least some of, the channels do not extend to any of the periphery of the absorbent material layer. In other words at least one of the channels of each type may be advantageously completely encompassed within the deposition area formed by the absorbent material. The channels in the core may in particular end at a distance of at least 5 mm from any outer edges of the periphery of the absorbent layer. The crotch channels and the front channels may be separated from each other by a separating zone or buffer comprising absorbent material. As represented in Figs. 5-8, the shortest distance d between the front channel and the crotch channel may be for example at least 5 mm, in particular from 5 mm to 20 mm. Keeping absorbent material for some distance between the front and crotch channels can help avoiding that fluid collected in the crotch channels are immediately conducted in the front channels, so that these front channels would prematurely disappear at the first gush of insulting liquid.

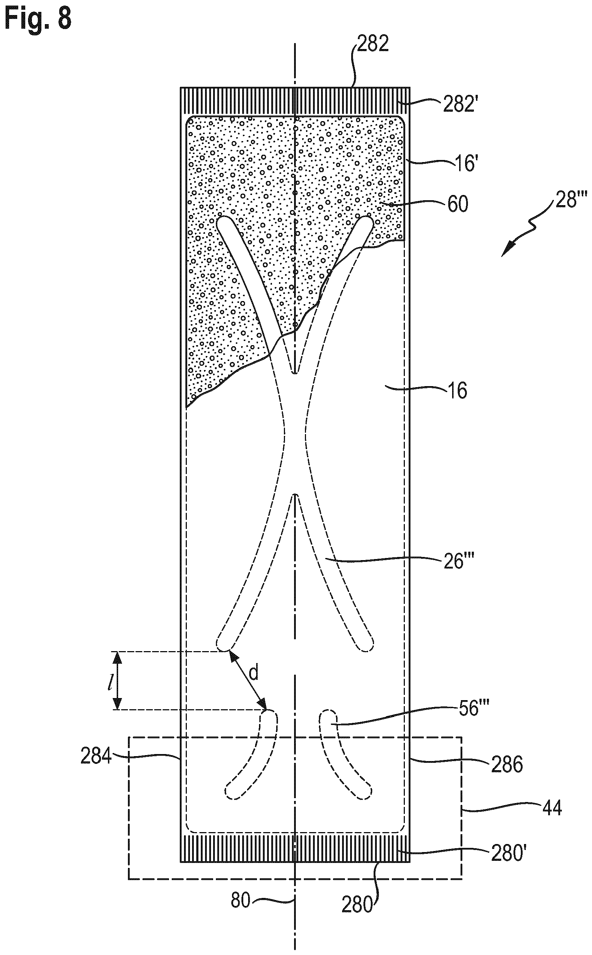

On the other hand, the longitudinal position of the front and crotch channels, as considered when projected on the longitudinal axis, may overlap, or be separated by a distance l between the front channel and the crotch channel ranging from 0 mm to less than 20 mm, or from 0 mm to less than 10 mm, or from 0 mm to less than 5 mm. In this way, the front channel may start at about the same longitudinal position where the crotch channel ends, so that the absorbent core comprises a channel from the front region to at least the crotch region and preferably the back region of the article without meaningful interruption. This continuous presence provides the benefits of the channels over an extended length of the absorbent core. The front and crotch channels may also partially overlap in the longitudinal direction. These distances l and d may also be higher, especially if relatively shorter crotch channels are used, as illustrated in Figs. 7 and 8.

Front and crotch channels may be respectively disposed as one or more pair of channels symmetrically disposed relative to the longitudinal axis, so that the crotch channels are mirror image of each other relative to the longitudinal axis, and likewise the front channels are mirror image of each other relative to the longitudinal axis. This typically provides for better fit and fluid distribution than if the channels were randomly or non-symmetrically disposed. Any pair of channels may be joined together along the longitudinal axis for example at their front and/or back extremities or in their center to form a X shaped channel as in Fig. 8 for example, or another symmetrical shape such as U or a V shape.

The crotch and front channels are typically longitudinally-extending, meaning that each channel extends at least as much in the longitudinal direction than in the transversal direction. The crotch channels typically extend more in the longitudinal direction than the front channels, for example at least twice as much in the longitudinal direction than the front channels (as measured as indicated previously after projection on the longitudinal axis). At least some the channels may have a width along at least part of their length which is at least 2 mm, or at least 3 mm or at least 4 mm, up to for example 20 mm, or 16 mm or 12 mm. The width for each channel may be constant through substantially the whole channel's length or may vary along its length. For example the channels may be straight and longitudinally oriented with the width gradually decreasing or increasing from the front to back of the article. The width of the channels may be the same or different between different channels.

The exemplary core shown in a top view in isolation on Fig. 5 has a pair of crotch channels 26 and a pair of front channels 56. Of course an absorbent core according to the invention may comprise more or less channels than represented in the illustrations. The absorbent core may for example also comprise a pair of channels in the back region 64 of the article.

The channels in Fig. 5 are curved. Some or all of the front and crotch channels may be in particular concave towards the longitudinal axis 80, as in inverted brackets ) (, in particular at least the crotch channels 26. Any of the channels may have an arcuate portion, and an angle between a tangent line of the arcuate portion and the longitudinal axis that is greater than or equal to 20 degrees. Channels with such an arcuate portion are for example disclosed in WO2014/093130A1 (Roe et al.). Of course different shapes and orientations for the front and/or crotch channels are possible. For example at least one of the front channels, and/or at least one of the crotch channels, or both channels, may be straight and parallel to the longitudinal axis, as exemplarily illustrated on Fig. 7. The channels may also be partially or entirely straight and oblique relative to the longitudinal axis. The channels may have other shape or orientation as those indicated above. For example the channels may be branched, or U shaped, and/or form a pocket towards the back of the article, especially for the bonded crotch channels, as exemplarily disclosed in WO2014/093129A (Roe et al.). There may or may not be a channel that partially extends longitudinally and coincides with the longitudinal axis of the article, however such a channel may create a folding line in the middle of the article that may cause the article to fold downwards which may cause some loss of contact.

When present as one or more symmetrical pair(s) relative to the longitudinal axis, the channels in each pair may be spaced apart from one another over part of or their whole longitudinal dimension. The spacing distance may be at some points or over the whole length of the channels for example at least 5 mm, or at least 10 mm, or at least 16 mm as measured in the transversal direction. Some channels are present as a pair may be also joined at their base as in a U-shape or V-shape, or may be joined towards their middle to form a cross or X-shape as illustrated in Fig. 8 for example.

## Upper substrate layer 16 and lower substrate layer 16'

The absorbent core 28 comprises an upper substrate layer 16 and a lower substrate layer 16' forming a core wrap which sandwiches the absorbent material. Various core wrap constructions are possible. The core wrap may in particular comprise, as represented in the Figures, two separate substrates 16, 16' forming the top side and the bottom side of the core wrap respectively. Having two different substrates for example allows more easily depositing an inner core glue on both the inner surface of the top side and the inner surface of the bottom side of the core wrap before combining these substrates to form the core wrap. The two substrates may be attached in a C-wrap or sandwich configuration with two longitudinal seals 284', 286', and optionally a front seal 280' and a back seal 282'. However this core wrap construction is not limiting of the invention, as any conventional core wrap construction may also be used, for example a single substrate on a portion of which the absorbent material is deposited and then the rest of the substrate folded over the deposited absorbent material to form the other side of the core. This single substrate construction can then be sealed longitudinally with a single longitudinal edge seal. The core wrap may also comprise two substrates disposed flat in a face to face relation (sandwich) with longitudinal side seals along their longitudinal sides.

The core wrap material may be any materials suitable for receiving and containing the absorbent material. Typical substrate materials used in the production of conventional cores may be used, in particular paper, tissues, films, wovens or nonwovens, or laminate of any of these. The core wrap may in particular be formed by a nonwoven web, such as a carded nonwoven, spunbond nonwoven ("S") or meltblown nonwoven ("M"), and laminates of any of these. For example spunmelt polypropylene nonwovens are suitable, in particular those having a laminate web SMS, or SMMS, or SSMMS, structure, and having a basis weight range of about 5 gsm to 15 gsm. Suitable materials are for example disclosed in US7,744,576, US2011/0268,932A1, US2011/0,319,848A1 and US2011/0,250,413A1. Nonwoven materials are typically made of synthetic fibers, such as PE, PET and in particular PP fibers. It is also possible than the core wrap may be at least partially formed from a component of the article having another function than merely serve as a substrate for the absorbent material. For example, it is possible that the backsheet may form the bottom side of the core wrap and/or that a distribution layer or the topsheet may form the top side of the core wrap. However, typically the core wrap is made of one or more substrates whose only or main function is to receive and enclose the absorbent material, as indicated previously.

As used herein, the terms "nonwoven layer" or "nonwoven web" generally means a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or synthetic origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter ($g/m^2$ or gsm).

As illustrated in Figs. 3-4, the upper substrate layer 16 may form substantially the whole of the top side of the core wrap and the lower substrate layer 16' may form substantially the whole of the bottom side of the core wrap, but it is not excluded that this may be the other way round. By "forming substantially the whole of the surface", it is meant that if present, the outwardly extending flaps of the other substrate that have been folded longitudinally may also form part of the surface considered. The first layer 16 may comprise two side flaps laterally extending along the length of the core and which are folded inwardly over each side edge 284, 286 of the absorbent core and the flaps may be attached to the outer surface of the second substrate 16' for example by using an adhesive seal along each C-wrap seal. One or two continuous or semi-continuous lines of glue may be typically applied along the length of the flaps to bond the inner surface of the flaps to the external surface of the other substrate. The reverse construction may of course also be used with the bottom substrate forming flaps over the top substrate.

The core may also comprise so-called sandwich seals where the lower and upper substrate layers are bonded along one edge of the core to each other in face-to-face relationship with the inner surface of each substrate bonded to the inner surface of the other substrate. These sandwich seals can for example be formed using a hotmelt glue applied in a series of stripes in a direction perpendicular to the front and back edges 280, 282 of the core. These end seals 280', 282' are however optional as many absorbent cores are left open at the front and back ends. The longitudinal edges may also be bonded by such a sandwich seal.

Having described in details the key features of the invention, the following sections provide more details on some of the typical components found in absorbent articles. The materials described below are of course optional and non-limiting, unless explicitly indicated otherwise.

**Inner core glue**

Having an inner core glue between the upper substrate layer and lower substrate layer of the core is advantageous. The inner core glue improves the adhesion of the absorbent material to the upper and/or lower substrate layer. The inner core glue may also at least partially form the bonds 27 between the substrates in the crotch channels and optionally the front channels. The inner core glue may also be responsible for weaker bonds 57 in the front channels, if these front channels have any bond at all. Both side of the core wrap may be locally pressed together within the areas of the crotch channels while the glue is still hot and fluid to increase the strength of the adhesive bonding in these areas.

The inner core glue may be applied on the inner surface of the upper and/or lower substrate on an area at least partially (e.g. at least 50 % and up to 100%) corresponding to the deposition area of the absorbent material to at least partially immobilize the absorbent material. The inner core glue may be applied according to any known techniques, in particular it may be applied as a series of longitudinally extending slots of glue as is known in the art, alternatively by other non-contact applicators such as spiral glue applicators, before the absorbent material is deposited on the nonwoven. The inner glue may thus be present in particular between the absorbent material and the inner surface of the bottom side of the core wrap, and/or between the absorbent material and the inner surface of the top side of the core wrap. An example of partial coverage of the deposition area by an inner core glue (also called auxiliary glue) to immobilize the absorbent material and to form channel bonds is for example disclosed in EP2,886,092 (Stelzig et al.). A fibrous thermoplastic material may also be present within the core wrap to help immobilizing the AGM particles, especially if the core is free of cellulose fibers.

**Topsheet 24**

The topsheet typically forms the majority of the wearer-contacting surface of the article and is the first layer that the body exudates contact. The topsheet is preferably compliant, soft-feeling, and non-irritating to the wearer's skin. Further, at least a portion of the topsheet is liquid permeable, permitting liquids to readily penetrate through its thickness. Any known topsheet may be used in the present invention. A suitable topsheet may be manufactured from a wide range of materials. Most topsheets are nonwoven materials or apertured formed films, but other materials are possible such as porous foams, reticulated foams, woven materials. Typical diaper topsheets have a basis weight of from about 10 gsm to about 28 gsm, in particular between from about 12 gsm to about 18 gsm but higher basis weights are possible if it is desired to provide a very soft feeling wearer-contacting surface for example.

Nonwoven topsheets may be made of natural fibers (e.g., wood or cotton fibers), synthetic fibers or filaments (e.g. polyester or polypropylene or bicomponent PE/PP fibers or mixtures thereof), or a combination of natural and synthetic fibers. If the topsheet includes nonwoven fibers, the fibers may be spunbond, carded, wetlaid, meltblown, hydroentangled, or otherwise processed as is known in the art. In particular the topsheet may be a spunbond PP nonwoven. A suitable topsheet comprising a web of staple-length polypropylene fibers is manufactured by Veratec, Inc., a Division of International Paper Company, of Walpole, MA under the designation P-8.

The topsheet may be of the type comprising a plurality of apertures. At least some of the apertures may have an area ranging from 1 mm$^2$ to 20 mm$^2$, and the topsheet may in particular comprise on average from 1 to 20 apertures per cm$^2$. The aperture ratio (the surface of all the apertures divided by the overall surface of the topsheet, measured when the topsheet is in a relaxed state, i.e. with just enough tension to smooth out any wrinkles) is advantageously in the range from 10% to 45%, in particular from 25% to 40%, more particularly from 30% to 35%. Typically, the total area of the apertures at the surface of a diaper may have an area of between about 10 cm$^2$ and about 50 cm$^2$, in particular between about 15 cm$^2$ and 35 cm$^2$. Examples of apertured topsheet are disclosed in US 6,632,504 (Gillespie et at.).

WO 2011/163582 (Rinnert et al.) also discloses a suitable colored nonwoven topsheet having a basis weight of from 12 to 18 gsm and comprising a plurality of bonded points. Each of the bonded points has a surface area of from 2 mm$^2$ to 5 mm$^2$ and the cumulated surface area of the plurality of bonded points is from 10 to 25% of the total surface area of the topsheet.

Suitable formed film topsheets are also described in US3,929,135, US4,324,246, US4,342,314, US4,463,045, and US5,006,394. Other suitable topsheets may be made in accordance with US4,609,518 and US4,629,643. Such formed films are available from The Procter & Gamble Company of Cincinnati, Ohio as "DRI-WEAVE" and from Tredegar Corporation, based in Richmond, VA, as "CLIFF-T". The topsheet may also have a three-dimensional appearance and feel, or there may be an additional, smaller, three-dimensional layer placed on top of the topsheet. Such three-dimensional additional layers may be for example particularly useful to receive low viscous exudates such as the stool of young babies Examples of such fluid entangled dual layered three-dimensional materials and processes to obtain them have been disclosed for example in US2014/0,121,623A1, US2014/0,121,621A1, US2014/0,121,624A1, US2014/0,121,625A1.

The topsheet may also be treated with a wetting agent to make it more hydrophilic. The wetting agent may be a surfactant as is known in the art. Other possible treatments are for example special coating by nanoparticles, as for example described in US6,645,569, US6,863,933, US2003/148684 and US2005/008839 (Cramer et al.) and US7,112,621 (Rohrbaugh et al). Any portion of the topsheet may also coated with a lotion as is known in the art. Examples of suitable lotions include those described in US5,607,760, US 5,609,587, US5,643,588, US5,968,025 and US6,716,441. The topsheet may also include or be treated with antibacterial agents, some examples of which are disclosed in WO95/24173. Further, the topsheet, the backsheet or any portion of the topsheet or backsheet may be embossed and/or matte finished to provide a more cloth like appearance.

### Backsheet 25

The backsheet may be any backsheet known in the art for absorbent articles. The backsheet may be positioned directly adjacent the garment-facing surface of the absorbent core. The backsheet prevents, or at least inhibits, the exudates absorbed and contained therein from soiling articles such as bedsheets and undergarments. The backsheet is typically impermeable, or at least substantially impermeable, to liquids (e.g., urine). The backsheet may, for example, be or comprise a thin plastic film such as a thermoplastic film having a thickness of about 0.012 mm to about 0.051 mm. The basis weight of those films is usually as low as possible to save material costs, typically from 10 gsm to 30 gsm, in particular below 20 gsm. A covering low basis weight nonwoven may be attached to the external surface of the film to provide for a softer touch.

Suitable backsheet materials include breathable materials which permit vapors to escape from the absorbent article while still preventing, or at least inhibiting, exudates from passing through the backsheet. Example breathable materials may include materials such as woven webs, nonwoven webs, composite materials such as film-coated nonwoven webs, microporous films such as manufactured by Mitsui Toatsu Co., of Japan under the designation ESPOIR NO and by Tredegar Corporation of Richmond, VA, and sold under the designation EXAIRE, and monolithic films such as manufactured by Clopay Corporation, Cincinnati, OH under the name HYTREL blend P18-3097.

The film may include at least about 10 weight percent filler particles, for example filler particles that include calcium carbonate, so that wherein the film has been stretched in the machine direction, e.g. to at least about 150 percent, fractures are formed where said filler particles are located. The films may be biaxially stretched at least about 150 percent in the machine direction and a transverse direction to cause fractures to form where said filler particles are located. Breathable films may generally have Water Vapor Transmission Rates (WVTR) in excess of 300 grams per square meter per 24 hours. The WVTR may be measured by the Desiccant Method as indicated in ASTM E96/E96M - 14.

US6,075,179 for example discloses a suitable multilayer film comprising: a core layer made from an extrudable thermoplastic polymer, the core layer having a first exterior surface and a second exterior surface, a first skin layer attached to the first exterior surface of said core layer to form the multilayer film, the multilayer film defining an overall thickness. The first skin layer defines a first skin thickness, and comprising less than about ten percent of said overall thickness. The overall thickness is not exceeding about 30 micrometers and the multilayer film is a liquid barrier and has a WVTR of at least 300 g/m2/24 hours.

The backsheet may further typically comprise a nonwoven on its most external side to improve softness. Exemplary laminates comprising a breathable film and a nonwoven layer are for example disclosed in WO2014/022,362A1, WO2014/022,652A1 and US5,837,352. The nonwoven web may in particular comprise a spunbond nonwoven web and/or a laminate of a spunbond nonwoven web and a meltblown nonwoven web. The laminate may also have a water vapor transmission rate of at least 300 g/m2/24 hours. US5,843,056 for example discloses substantially liquid impermeable, vapor permeable composite backsheet.

### Front and back ears 46, 40

The absorbent article typically comprises front ears 46 and back ears 40 as is known in the art in taped diapers. The ears can be integral part of the chassis, for example formed from the topsheet and/or backsheet as side panel. Alternatively, as represented in Fig. 1, they may be separate elements attached by gluing and/or heat embossing. The back ears 40 are preferably stretchable to facilitate the attachment of the fastening tapes 42 to the landing zone 44, and maintain the taped diapers in place around the wearer's waist. The front ears 46 may be optionally elastic or extensible to provide a more comfortable and contouring fit.

### Barrier leg cuffs 34 and gasketing cuffs 32

The absorbent articles may typically further comprise cuff components 30 that improve the fit of the article around the legs of the wearer. Such cuffs typically comprise barrier leg cuffs 34 and gasketing cuffs 32. The cuffs 30 may comprise a piece of material, typically a nonwoven, which is one side partially bonded to the article and on the other side can be partially raised away from the topsheet and thus stand up from the plane defined by the topsheet as shown for example in Fig. 3. Both parts of the cuffs may be advantageously elasticized. The raised part of the cuff components is referred to herein as barrier leg cuffs 34 and can provide improved containment of liquids and other body exudates approximately at the junction of the torso and legs of the wearer. The barrier leg cuffs 34 extend at least partially between the front edge and the back edge of the absorbent article on opposite sides of the longitudinal axis and are at least present adjacent to the center point C of the article.

The barrier leg cuffs 34 may be delimited by a proximal edge 37 joined to the rest of the article, typically the topsheet, and a free terminal edge 38 intended to contact and form a seal with the wearer's skin. The barrier leg cuffs 34 may be joined at the proximal edge 37 with the chassis of the article by a bond which may be made for example by adhesive bonding, fusion bonding or combination of known bonding means, for example as disclosed in WO2014/168,810A1 (Bianchi et al.). The bond at the proximal edge 37 may be continuous or intermittent.

The barrier leg cuffs 34 can be integral with (i.e. formed from) the topsheet or the backsheet, or more typically be formed from a separate material joined to the rest of the article. Typically the material of the barrier leg cuffs may extend through the whole length of the article but is "tack bonded" to the topsheet towards the front edge and back edge of the article so that in these sections the barrier leg cuff material remains flush with the topsheet. Each barrier leg cuff 34 may comprise one, two or more elastic strings 35 close to its free terminal edge 38 to provide a better seal.

In addition to the barrier leg cuffs 34, the article may comprise gasketing cuffs 32, which are formed in the same plane as the chassis of the absorbent article, in particular may be at least partially enclosed between the topsheet and the backsheet, and typically placed further laterally outwardly relative to the barrier leg cuffs 34. The gasketing cuffs 32 can provide a better seal around the thighs of the wearer. Usually each gasketing leg cuff 32 will comprise one or more elastic string or elastic element 33 comprised in the chassis of the diaper for example between the topsheet and backsheet in the area of the leg openings. Typically the barrier leg cuffs 34 are disposed more internally than the gasketing cuffs 32. The barrier leg cuffs are thus also referred to as inner cuffs and the gasketing cuffs as outer cuffs.

For example, US3,860,003 describes a disposable diaper which provides a contractible leg opening having a side flap and one or more elastic members to provide an elasticized leg cuff (a gasketing cuff). US4,808,178 (Aziz) and US4,909,803 (Aziz) describe disposable diapers having "stand-up" elasticized flaps (barrier leg cuffs) which improve the containment of the leg regions. US4,695,278 (Lawson) and US4,795,454 (Dragoo) describe disposable diapers having dual cuffs, including gasketing cuffs and barrier leg cuffs. All or a portion of the barrier leg and/or gasketing cuffs may be treated with a lotion.

**Acquisition layer 52**

The absorbent article may advantageously comprise an acquisition layer 52, sometimes referred to as secondary topsheet, whose function is to quickly acquire the fluid away from the topsheet so as to provide a good dryness for the wearer. The acquisition layer is typically placed directly under the topsheet. There may be optionally a distribution layer (not represented) at least partially disposed under the acquisition layer 52. The acquisition layer may typically be or comprise a non-woven material, for example a SMS or SMMS material, comprising a spunbonded, a melt-blown and a further spunbonded layer, but many other alternatives material are known in the art and may be used instead in particular a cared nonwoven. Nonwovens have the advantage that they can be manufactured outside the converting line and stored and used as a roll of material. The nonwoven material may be latex bonded. Exemplary upper acquisition layers are disclosed in US7,786,341. Carded, resin-bonded nonwovens may be used, in particular where the fibers used are solid round or round and hollow PET staple fibers (50/50 or 40/60 mix of 6 denier and 9 denier fibers). An exemplary binder is a butadiene/styrene latex. Further useful nonwovens are described in US6,645,569 (Cramer et al.), US6,863,933 (Cramer et al.), US7,112,621 (Rohrbaugh et al.), US2003/148684 (Cramer et al.) and US2005/008839 (Cramer et al.). The acquisition layer may be stabilized by a latex binder, for example a styrenebutadiene latex binder (SB latex). Processes for obtaining such latices are known, for example, from EP 149880 (Kwok) and US 2003/0105190 (Diehl et al.). The binder may typically be present in the acquisition layer in amount ranging from about 12% to about 50%, for example about 30%, by total weight of the acquisition layer. SB latex is available under the trade name GENFLO™ 3160 (OMNOVA Solutions Inc.; Akron, Ohio).

Another typical acquisition layer may be a bonded carded web, in particular a through-air bonded carded web ("TABCW"). "Bonded carded web" refers to webs that are made from staple fibers that are sent through a combing or carding unit, which breaks apart and aligns the staple fibers in the machine direction to form a generally machine direction-oriented fibrous nonwoven web. This web is then drawn through a heated drum, creating bonds throughout the fabric without applying specific pressure (thru air bonding process). A TABCW material provides a low density, lofty through-air bonded carded web. The web may for example have a specific weight basis level at about 15 gsm to about 120 gsm (gram per square meter), in particular about 30 gsm to about 80 gsm. A TABCW material can for example comprise about 3 to about 10 denier staple fibers. Examples of such TABCW are disclosed in WO2000/71067 (KIM DOO-HONG et al.). TABCW are available directly from all usual suppliers of nonwoven webs for use in absorbent articles, for example Fitesa Ltd or Fiberweb Technical Nonwovens.

A further acquisition layer (not shown) may be used in addition to the first acquisition layer described above. For example a tissue layer may be placed between the first acquisition layer and a distribution layer. The tissue may have enhanced capillarity distribution properties compared to the acquisition layers described above. The tissue and the first acquisition layer may be of the same size or may be of different size, for example the tissue layer may extend further in the back of the absorbent article than the first acquisition layer. An example of a hydrophilic tissue is a 13 to 15 gsm high wet strength tissue made of cellulose fibers from supplier Havix.

**Distribution layer**

The article may comprise a further intermediate layer (not represented) between the topsheet and the absorbent core, which will be referred herein as a distribution layer. The function of a distribution layer is to spread an insulting fluid liquid over a larger surface within the article so that the absorbent capacity of the core can be more efficiently used. Such a distribution layer may be smaller in surface than the absorbent core's footprint and does typically not extend beyond the edges of the core's footprint. The distribution layer is typically made of a fibrous material, which may be based on synthetic or cellulosic fibers. The distribution layer may also comprise channels that may at least partially or completely match the positions and the shape of all or any of the channels of the absorbent core. The same consideration regarding the shape, position and orientation for the channels of the absorbent core can be re-applied for channels a distribution layer and thus will not be repeated herein.

The distribution layer may thus be a fibrous layer which has an average basis weight of at least 50 g/m$^2$, in particular from 50 g/m$^2$ to 300 g/m$^2$, and advantageously at least at least 100 g/m$^2$. The average basis weight is calculated by dividing the weight amount of the fibers by the area of the distribution where the fibers are present (including channel area in the distribution layer). The distribution layer may have a relatively low density. The density of the layer may vary depending on the compression of the article, but may typically range from 0.03 g/cm$^3$ to 0.25 g/cm$^3$, in particular from 0.05 g/cm$^3$ to 0.15 g/cm$^3$, measured at 0.30 psi (2.07 kPa). The density of the intermediate layer is measured at the centerpoint C of the article for this purpose.

The fibrous material may be manufactured by air-laying the fibers on a drum comprising several molds each having the required depth profile for the desired fibrous material configuration. The formed distribution layer can then be directly un-molded onto another component of the article such as a nonwoven carrier layer and then integrated with the rest of the article. There may be other layers between the distribution layer and any of the topsheet and the absorbent core, for example an acquisition layer 52. When a nonwoven acquisition layer is present in the article, the distribution layer may be for example deposited on this acquisition layer, the two layers being further joined to absorbent core and the rest of the article, as is known in the art.

The distribution layer is typically a fibrous layer. The distribution layer may be a nonwoven material comprising fibers that are bonded to each other so that the layer has a strong integrity and may be manipulated independently of a substrate. Alternatively, the distribution layer may be another type of fibrous layer, in particular the distribution layer may comprise or consist of loose fibers with no or weak intra-fiber bonds, the fibers being deposited on a supporting substrate at varying basis weight to form a profiled distribution. A typical example of distribution/intermediate material comprises or consists of cross-linked cellulose fibers. The distribution/intermediate layer may for example comprise at least 50% by weight of cross-linked cellulose fibers. The cross-linked cellulosic fibers may be crimped, twisted, or curled, or a combination thereof including crimped, twisted, and curled. This type of material has been used in the past in disposable diapers as part of an acquisition system, for example US 2008/0312622 A1 (Hundorf). The cross-linked cellulosic fibers provide higher resilience and therefore higher resistance against the compression in the product packaging or in use conditions, e.g. under baby weight.

Exemplary chemically cross-linked cellulosic fibers suitable for a distribution layer are disclosed in US5,549,791, US5,137,537, WO95/34329 or US2007/118087. The distribution layer comprising cross-linked cellulose fibers may comprise other fibers, but this layer may comprise at least 50%, or 60%, or 70%, or 80%, or 90% or even up to 100%, by weight of the layer, of cross-linked cellulose fibers (including the cross-linking agents). While the distribution material may be comprised of cellulose fibers, in particular cross-linked cellulose fibers, other materials are possible.

**Lower acquisition and distribution layer (ADS)**

The absorbent article may comprise a lower acquisition and distribution layer, herein referred to as "lower ADS". The lower ADS serve as a temporary reservoir for liquid that has flown through the layer of absorbent material because it was not absorbed fast enough by the absorbent material. The lower ADS is disposed between the absorbent material layer and the fluid-impermeable backsheet. The lower ADS may be either a single layer, thus also taking the role and function of the lower substrate layer 16', or the lower ADS may comprise several layers, such as a standard low basis weight lower substrate layer 16' and at least one additional lofty layer disposed between the lower substrate layer 16' and the backsheet.

The lower ADS preferably comprises a layer of a relatively lofty material, providing sufficient void volume to acquire and hold a fluid that penetrates through the layer of absorbent material. The lower ADS may comprise a masking layer material as disclosed for example in WO2019/241,009A1 (P&G, Tally et al.). The lofty layer is typically free of superabsorbent polymer. At the same time, the lofty layer preferably provide softness and do not have an excessively high bending stiffness. Such layer may in particular be a spunlace, an air-through bonded nonwoven, or a crimped fiber spunbond nonwoven.

The lofty layer may comprise continuous fibers, such as in a spunlaid nonwoven web. The spunlaid nonwoven web is preferably air-through bonded or spunlace. In addition to hydroentanglement (spunlace) or air-through bonding, the spunlaid nonwoven web may or may not have undergone some localized bonding with heat and/or pressure (e.g. point bonding), introducing localized bond regions where the fibers are fused to each other.

Preferably, the lofty layer comprises staple fibers. Similar to a nonwoven web made of continuous fibers, a nonwoven web of staple fibers is preferably air-through bonded or spunlace. In addition to hydroentanglement (spunlace) or air-through bonding, the nonwoven web of staple fibers may or may not have undergone some localized bonding with heat and/or pressure (e.g. point bonding), introducing localized bond regions where the fibers are fused to each other.

Irrespective whether the lofty web is made of continuous fibers or staple fibers, the localized bonding should however not bond an excessively large surface area, thus negatively impacting the loft and void volume of the nonwoven web. Preferably, the total bond area obtained by localized bonding with heat and/or pressure (in addition to hydroentanglement or air-through bonding) should not be more than 20%, or not be more than 15%, or not be more than 10% of the total surface area of the lofty web. Alternatively, the lofty nonwoven web comprised by the lower ADS should not have undergone any bonding and consolidation in addition to the hydroentanglement (spunlace) or air-through bonding. Thereby, the advantageous properties of such nonwoven webs can be used to their optimum.

Additional layers provided to an absorbent article generally increase the thickness and bulk of the article. This may lead to increased bending stiffness, and thus to drawbacks for conformity and close contact of the article to the wearer's body, thereby reducing wearer comfort. Also, increased bulk is generally not desirable, especially between the wearer's legs. According to the invention, the presence of the longitudinally-extending crotch and front channels however provide improved bending for the absorbent article, so that any of these drawbacks can be mitigated. The articles of the present invention may thus be particularly suitable for being provided with a lower ADS, in particular a lower ADS having a relatively high basis weight.

The absorbent article may thus optionally comprise a lower ADS disposed between the absorbent material layer and the backsheet. Such a lower ADS has typically a higher basis weight than a typical core substrate layer which is around 10 $g/m^2$. The basis weight of the lower ADS, which may consist of a single layer or a combination of two or more layers, may typically range

from 20 $g/m^2$ to 120 $g/m^2$, or from 25 $g/m^2$ to 110 $g/m^2$, or from 30 $g/m^2$ to 100 $g/m^2$. The basis weight of the lower ADS is calculated by adding the basis weight of each layer disposed between the absorbent material layer and the backsheet. Typically, this basis weight may be the basis weight of the lofty lower substrate layer 16' for a single layer lower ADS, or the lower substrate layer 16' and one (or more) additional lofty layer disposed between the lower substrate layer 16' and the backsheet. The basis weight of the layer(s) comprised in the lower ADS are typically homogeneous and indicated by the supplier, but if not the average basis weight can be calculated simply by dividing the weight of each layer by their area.

The lower ADS comprises preferably at least one layer having a lofty structure. The use of crimped fibers may be beneficial to form a lofty layer. Such fibers have also shown to provide the nonwoven layer with good resiliency, i.e. the nonwoven web has a relatively good ability to regain its original caliper (or most of its original caliper) after it has been compressed for a longer time (e.g. while being contained in a closed package that contains highly compressed absorbent articles). The crimped fibers may have flat crimp (so-called two-dimensional crimp) or three-dimensional crimp, such as spiral crimp. Bicomponent fibers are well known as being suitable for obtaining crimped fibers. The lower ADS may thus comprise at least one layer having a 30 weight-%, or at least 40 weight-%, or at least 50 weight-%, or at least 70 weight-%, or at least 90 weight-% or 100 weight-% of crimped fibers based on the total weight of the layer as part of the lower ADS. The crimped fibers may be bicomponent fibers.

The caliper of the lower ADS is desirably in a range that balances good liquid absorption and liquid holding properties (i.e. sufficient void volume within the nonwoven web) with the need to avoid that the lower ADS adds excessive bulk to the absorbent article, thus decreasing wearer comfort. The caliper of the lower ADS may be from 0.1 to 2.0 mm, or from 0.2 to 1.0 mm, as measured according to the test method set out herein below. If the ADS comprises more than one layer, the caliper of each layer may be measured separately and added together to report the caliper of the lower ADS.

Also, the lower ADS desirably comprise at least one layer exhibiting good recovery after compression, given absorbent articles are often packed under relatively high compression. A material that initially had suitable characteristics for use as lower ADS, e.g. sufficient loftiness and void volume, may lose much of these beneficial properties upon compression in the packaging if its ability to recover is insufficient. This ability is reflected by the Z-Compliance Index and Percent Recovery Measurement Method set out below. The lower ADS may consist of or comprise a layer having a Compliance Index greater than 4, preferably greater than 10, and a Percentage Recovery greater than 50%, preferably greater than 60%.

The nonwoven comprised by or forming the lower ADS may have undergone mechanical deformation. Such mechanical deformation can contribute to the loft and openness of the nonwoven web, hence improving those properties of the nonwoven web which are desirable for use as lower ADS. The lower ADS may comprise a layer having a three-dimensional surface topography (as may, for example, be obtained by mechanical deformation), so-called "air pockets".

To not unduly increase the stiffness of the absorbent article, the layer or layers lower ADS may have a Horizontal Bending Drop according to the test method set out below, of greater than 60, preferably greater than 70. Higher values for the Horizontal Bending Drop indicate that a material is more flexible versus lower values.

**Other components**

The absorbent articles of the invention can further comprise any other typical components known for the intended purpose of the article that are not illustrated in the Figures, such as a transverse barrier element extending across the topsheet to form a receptacle for bowel movement, a lotion application on the topsheet, a wetness indicator comprising a pH indicator disposed between the absorbent core and the backsheet, etc. These components are well-known in the art and will not be further discussed herein. Reference is made to WO2014/093310 where several examples of these components are disclosed in more details.

The absorbent article may also comprise at least one elastic waist band (also called elastic waist feature) disposed parallel to and along the back edge of the article and less commonly parallel to and along the front edge of the article. Such waistbands help providing improved fit and containment at the back and/or front edge of the article. The elastic waist feature is generally intended to elastically expand and contract to dynamically fit the wearer's waist. The elastic waist band may be constructed in a number of different configurations. Non-limiting examples of back and front waistbands can be found in WO2012/177,400 and WO2012/177,401 (Lawson), and US4,515,595, US4,710,189, US5,221,274 and US6,336,922 (VanGompel et al.).

**Packages**

A plurality of articles according to the invention may be packaged in a package for transport and sale. At least 50% of the articles, and preferably all the articles, in the package may be according to the invention. The articles may be folded and packaged as is known in the art. The package may be for example a plastic bag or a cardboard box. Diapers may typically bi-folded along the transversal axis and the ears folded inwardly before being packaged. The absorbent articles may be packed under compression so as to reduce the size of the packages, while still providing an adequate amount of absorbent articles per package. By packaging the absorbent articles under compression, caregivers can easily handle and store the packages, while also providing distribution and inventory savings to manufacturers owing to the size of the packages.

The absorbent articles may thus be packaged compressed at an In-Bag Compression Rate of at least 10%, in particular of from 10% to 50%, in particular from 20% to 40%. The "In-Bag Compression Rate" as used herein is one minus the height of a stack of 10 folded articles measured while under compression within a bag ("In-Bag Stack Height") divided by the height of a stack of 10 folded articles of the same type before compression, multiplied by 100; i.e. (1-In-Bag Stack Height/stack height before compression) * 100, reported as a percentage. Of course, the stack in the bag does not need to have exactly 10 articles, rather the value measured for the height of stack of article in the package is divided by the number of articles in the stack and then multiplied by 10. The method used to measure the In-Bag Stack Height is described in further details in the Test Procedures. The articles before compression are sampled from the production line between the folding unit and the stack packing unit. The stack height before compression is measured by taking 10 articles before compression and packing, and measuring their stack height as indicated for the IBSH.

Packages of the absorbent articles of the present disclosure may in particular have an In-Bag Stack Height of less than 110 mm, less than 105 mm, less than 100 mm, less than 95 mm, less than 90 mm, specifically reciting all 0.1 mm increments within the specified ranges and all ranges formed therein or thereby, according to the In-Bag Stack Height Test described herein. For each of the values indicated in the previous sentence, it may be desirable to have an In-Bag Stack Height of greater than 60, or greater than 70 mm, or greater than 75 mm, or greater than 80 mm. Alternatively, packages of the absorbent articles of the present disclosure may have an In-Bag Stack Height of from 60 mm to 110 mm, from 75 mm to 110 mm, from 80 mm to 110 mm, from 80 mm to 105 mm, or from 80 mm to 100 mm, specifically reciting all 0.1 mm increments within the specified ranges and all ranges formed therein or thereby, according to the In-Back Stack Height Test described herein.

**Process for making**

The topsheet 24, the backsheet 25, the absorbent core 28, the acquisition layer 52 and the other article components may be assembled in a variety of well-known configurations, in particular by gluing, heat-, ultrasonic- and/or pressure- bonding as is known in the art. Typically, adjacent layers will be joined together using conventional bonding method such as adhesive coating via slot coating or spraying on the whole or part of the surface of the layer, or thermo-bonding, or pressure bonding or combinations thereof. Most of the bonding between components is for clarity and readability not represented in the Figure. Bonding between the layers of the article should be considered to be present unless specifically excluded. Adhesives may be typically used to improve the adhesion of the different layers, for example between the backsheet and the core wrap. The adhesives used may be any standard hotmelt glue as known in the art. The individual components may be converted into an absorbent article according to any of the processes known in the art. These bonds are typically not represented in the Figures to preserve readability of the Figures, but are present as is known in the art.

If the article comprises an acquisition or distribution layer comprising material free channels, the topsheet can be attached directly or indirectly to the absorbent core through these channels. The topsheet may thus be bonded to the top side of the absorbent core through channels in the acquisition or distribution layer, for example by adhesive bonding (gluing). Indirect bonding of the topsheet to an underlying layer may also be provided when an acquisition layer not comprising channels is present between the topsheet and a distribution layer with channels.

According to an aspect of the invention, a process for making the absorbent article may comprise the following steps of:

> i) providing an upper substrate layer and a lower substrate layer;
> ii) optionally applying an adhesive on the upper substrate layer and / or lower substrate layer;
> iii) depositing an absorbent material on at least one of the upper substrate layer or lower substrate layer to form an absorbent layer, wherein the absorbent layer comprises at least one crotch channel and a front channel, the channels being substantially free of absorbent material, and if an adhesive has been applied, this adhesive is present between the absorbent layer and the upper substrate layer and / or lower substrate layer;
> iv) bonding the upper substrate layer and the lower substrate layer to each other through the crotch channel by a crotch channel bond and optionally through the front channel by a weaker front channel bond, to form an absorbent core;
> v) forming one or more core wrap seals (280', 282', 284', 286') to obtain an absorbent core;
> vi) assembling the absorbent core thus obtained with the other absorbent article's components including a landing zone material so that the front channel is at least partially superposed with the landing zone.

The process may advantageously comprise the step of applying an inner core glue on the inner surface of the upper substrate layer or lower substrate layer so that at least a portion of the absorbent material area is adhesively immobilized on the top side and/or the bottom side of the core wrap.

The inner core glue may be typically on at least one, or both, of the inner surface of the top side and/or the bottom side of the core wrap to provide for better immobilization of the absorbent material. The inner core glue may also form or at least contribute to the bonds of the crotch channels (and optionally the front channels as well, as long as the front channel bonds are weaker). The inner core glue may be applied as a pattern of longitudinally-extending stripes, or spirals, or any other pattern as is known in the art. The inner core glue may be applied on the core wrap material before the absorbent material is deposited thereon, or alternatively on the second part of the core wrap that is folded over, or separately added to the core wrap material.

The upper substrate layer and lower substrate layer can be, as indicated before, any usual material known in the art, typically a nonwoven. The absorbent material may be deposited as a layer on the core wrap material using any known suitable techniques. The deposition may be continuous, for example as in an airlaying process where a constant flow of particles and cellulose fibers are mixed in a chamber before being pulled by negative pressure towards the core wrap material on the other side of the airlaying chamber. The substrate upper or lower layer may typically lay on a rotating drum while the absorbent material is deposited with the airlaying chamber being stationary. The outer surface of the drum comprises raised portions matching the shapes of the desired channels so that that substantially no absorbent material is deposited in these areas. Alternative processes for deposition the absorbent material while leaving channel areas substantially free of absorbent material include those processes used in airfelt-free absorbent cores, under the general description of AGM printing, are generally disclosed in EP2,532,329, EP2,905,000A1, and EP2,905,001 (all Jackels et al.).

The upper substrate layer and the lower substrate layer of the core are bonded to make one or more channel bonds 27, as indicated above. The crotch channel bond may be in particular provided by an inner core glue and an additional bonding means, in particular wherein the additional bonding means is a reinforcing glue and/or ultrasonic bonding and/or thermo bonding and/or additional pressure on the adhesive bond between the upper and lower substrate layers. The front channels may be completely unbonded, or if they comprise a weaker bond this bond may be in particular limited to a bond provided for example by the same inner core glue as the crotch channel of bonds, but do not comprise an additional bonding means as indicated above. For example, the crotch channel bonds may be adhesive bonds wherein the top side and the bottom side of the core wrap have been locally pressed together to increase the strength of the adhesive bonds, while the core wrap layers in front channels have not been pressed together, and thus do not form a strong bond even if they comprise some of the inner core glue. In another example, the crotch channel bond may be an adhesive bond complemented with an additional bonding means such as a thermo- or ultrasonic bond, and the front channel bond may comprise no bond or only the same type of adhesive bond as the crotch channel bonds without the additional bonding means.

## Test procedures

The values indicated herein are measured according to the methods indicated herein below, unless specified otherwise. All measurements are performed at 21°C ± 2°C and 50% ± 5% RH, and samples should be kept at least 24 hours in these conditions to equilibrate before conducting the tests, unless indicated otherwise. For example the Static Peel Force Time is measured at 23 degrees as is indicated below.

## Static Peel Force Time

The purpose of the Static Peel Force Time (SPFT) test method is to measure the bond strength between the top side and the bottom side of the core wrap within the channels. This test method measures how long the bond is able to withstand a constantly applied vertical force of about 150 grams ( Static Peel Force Time ) under standardized conditions.

Equipment

| | |
|---|---|
| Medium Clip | Medium Binder Clips 25 mm Capacity (#72050). ACCO World Product. Other suppliers: Yihai Products (#Y10003), Universal Office Products (#10210), Diamond (#977114), or equivalent. The clip weights 4.5 g +/-1 g. |
| Large Clip | Large Binder Clips 2 inch (50.8mm). ACCO World Product. Other suppliers: Yihai Products, Universal Office Products, Diamond, or equivalent |
| Test Stand | RT-10 room temperature (Shear Tester) w/ timer. ChemInstruments, 510 Commercial Drive, Fairfield Ohio 45014-9797, USA; or equivalent. Must be placed in a vibration free area. |
| Weight | 150g (+/- 1g) TW150 Shear Tester Weight with hook on top (to attach to the clip). ChemInstruments, 510 Commercial Drive, Fairfield Ohio 45014-9797, USA; or equivalent |
| Cutting Tools | Scissors and a 25.4 mm (1 inch) cutter (convenient source, e.g JDC Precision Sample Cutter made by Thwings-Albert Instrument Company Philadelphia USA, cat# 99, cut width 25.4 mm, accuracy at least +/- 0.1 mm) |
| Metal Ruler | Traceable to NIST, DIN, JIS or other comparable National Standard, graduated in mm, longer than the length to be measured |
| Temperature | Testo-temperature device (or equivalent) to measure temperature at sample height with an accuracy of ± 0.5 °C and ± 2.5 % RH in the range between -10°C and +50°C. Testo GmbH & Co., Postbox 1140, D-79849 Lenzkirch (www.testo.com) Article number for Testo 625: 0563 6251. |

Core preparation:
Typically the absorbent core is taken from a commercial article (e.g. diaper), and can be extracted from the article as follows:

1. Open the diaper topsheet side up and place it flat onto a table. Hold the diaper with one hand and carefully remove the ears (40) and the barrier cuffs (30) along the cuffs continuous bond (outer edge) on both sides of the articles.
2. Gently remove topsheet and acquisition system without damaging the core end seals if present. The backsheet does not need to be removed.

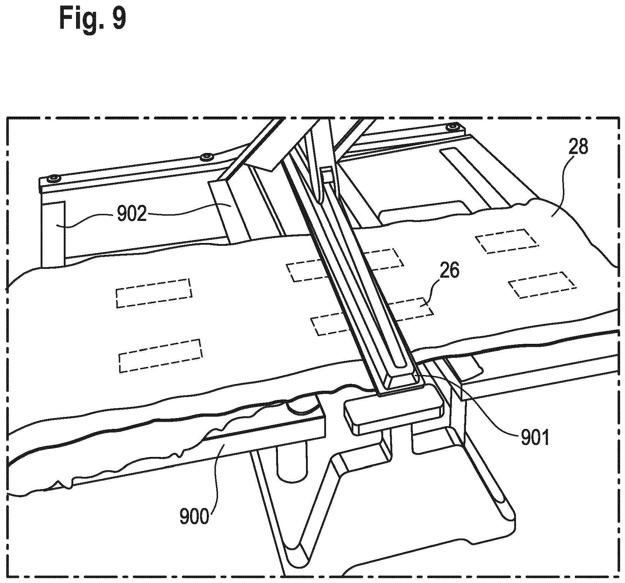

Channel Sample preparation (Fig. 9):

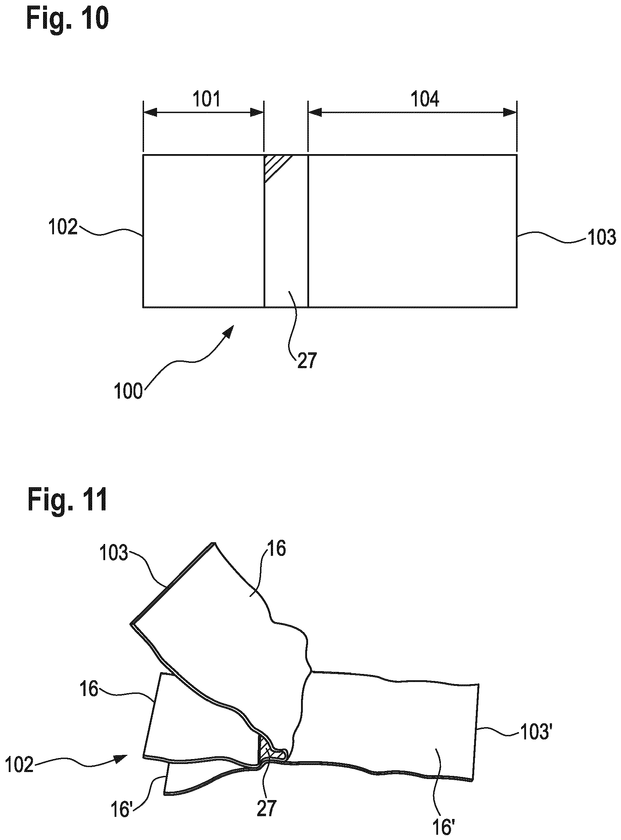

1. Put each core under a lamp table or a UV-light to identify the beginning and the end of each channel.
2. Define the longitudinal center of the channel area by using a ruler and mark a line perpendicular to the longitudinal axis passing through the center of the channel area.
3. Lay the core (28) on a supporting table (900) fitted with the 25.4 mm wide cutter (901) and align to the centerline of the channel. Double sided tapes (902) may be used to keep the sample in place. The table comprises two grooves or gaps on each side of the area to be cut so that the cutter blade can penetrate through the thickness of the core.
4. Cut the core in transversal direction to obtain a sample band centered on the centerline of the channel, optionally check the width of the cut sample band (target = 25 ± 2 mm).
5. Use the scissors to cut the 1-inch wide sample band in the longitudinal direction to obtain a sample (100) comprising the channel bond (27). There should be at least a 5 mm channel-free flap (101) between the channel bond and the inner edge (102) of the sample to ensure proper manipulation of the sample- see Fig. 10. Obtain a left sample and right sample if applicable. Label the cut samples appropriately, e.g. left/right channels.
6. The sample will be clamped on the outer edge (103) during the test. The outer edge (103) of the sample can be trimmed with the scissors, however in order to carry the test, the minimum channel distance (104) to the outer edge (103) for the sample should be sufficient to ensure a proper clamping of the core wrap material into the clamps. Typically a minimum distance (104) between the channel and the outer edge should be of at least 5 mm for this purpose, 20 mm being ideal.

7. Gently remove any core absorbent material outside the channel that is between bottom side and the top side of the core wrap (see Fig. 11), for this open any core wrap longitudinal side deals on the outer edge (103) if needed in case the seals was trimmed away already.

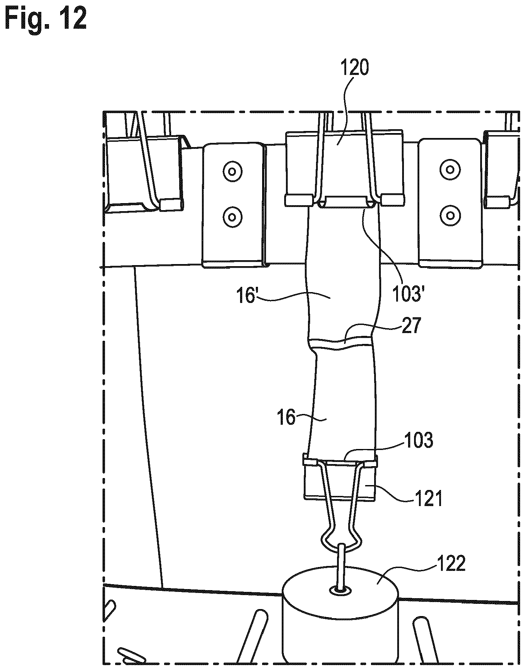

Test Procedure (see Fig. 12):

Set up the tester in an area where the temperature is constant at 23°C and ensure that the tester and the samples have at least 2 hour time to reach this temperature.

1. Clamp the outer edge (103') of the bottom side (16') of the sample into the jaw of the large binder (120) clip hanging at the top of the tester bar.

2. Clamp the other binder clip (medium size) (121) to the top side (16) of the core wrap at its outer edge (103). The inner edge (102) of the sample is facing away from the experimenter.

3. Slowly attach the 150 g weight (122) to the medium binder clip and lower slowly until the weights hangs freely on the test sample.

4. As soon as the weight is released, push the timer reset button for that sample to begin the timer at 0 minutes. NOTE: The timer must be checked to ensure that it has begun counting from 0.0 min. The operator should look for the number to change from 0.0 min to 0.1 min.

5. Repeat procedure above for each sample prepared. The Test Stand allows testing several samples in parallel.

6. The timers will stop once the sample weight has fallen on the bottom plate due to the bond breaking. The bottom plate comprises a switch linked to the timer so that it automatically stops when the weight has fallen down. The time recorded is the Static Peel Force Time for that sample (expressed in minutes). Note: if the weight falls down due to the sample slipping out of the clip (121) without the bond breaking, the test needs to be rerun on a new sample.

7. If the sample weight has not fallen after 999 minutes, the Static Peel Force Time is reported to be 999 minutes (maximum Static Peel Force Time Time).

8. For a commercial article of a given construction, the experiment is repeated on 10 different samples extracted from 10 individual articles or cores, for example 10 randomly selected diapers in a commercially-sourced diaper package, and the result averaged. The Static Peel Force Time is this average.

**Centrifuge Retention Capacity (CRC)**

The CRC measures the liquid absorbed by the superabsorbent polymer particles for free swelling in excess liquid. The CRC is measured according to EDANA method WSP 241.2.R3 (12).

**Caliper test method**

The Caliper of the lower ADS is determined using the Caliper Test Method. In the Caliper Test Method, two flat, parallel surfaces are used to apply unidirectional pressure to both sides of a substrate specimen, and the resulting separation between the parallel surfaces is measured. All measurements are performed in a laboratory maintained at $23 \pm 2$ °C and $50 \pm 2$ % relative humidity and test specimens are conditioned in this environment for at least 2 hours prior to testing. If the lower ADS comprises two or more layers, the procedure is repeated for each constituent layer and the intermediate results added to report the combined caliper of the lower ADS.

Two parallel circular surfaces of 5.6 cm diameter are oriented horizontally. If possible, measurements are made on the sample material before it is integrated in an absorbent article. If this is not possible, care should be exerted when excising the sample material from the product not to impart any contamination or distortion to the sample layer during the removal of the sample layer from other layers (using cryogenic spray, such as Cyto-Freeze, Control Company, Houston, Texas, if needed). Five equivalent rectangular specimens are taken from the material of five products such that each specimen center corresponds to the position of the center point C of the article. The length and width of each specimen is greater than 5.6 cm. A specimen is then placed between the two parallel circular surfaces so that it completely covers each of the parallel surface and such that the center of the material specimen is matching with the center of the parallel circular surfaces.

The parallel surfaces are then brought together at a rate of $3.0 \pm 1.0$ mm/s until a pressure of 0.3 psi (2.1 kPa) is achieved, and the separation between the plates is measured and recorded to the nearest 0.01 mm within 2 seconds. The arithmetic mean of the plate separation of the 5 individual replicate specimens is calculated and reported as the Caliper of the substrate under 2.1 kPa in units of millimeters (mm) to the nearest 0.01 mm.

One suitable example of apparatus for use in the Caliper Method is a Mitutoyo Digimatic Series 543 ID-C digital indicator (Mitutoyo America Corp., Aurora, Illinois, USA), or equivalent, fitted with a circular flat "foot" at the end of the moving shaft of the indicator gauge. The indicator is mounted on a horizontal granite base such that the shaft of the indicator gauge is oriented vertically, and the plane of the circular foot is parallel to the granite base. The circular foot is sized and weighted such that the gravitational force associated with the mass of the foot and the indicator shaft together divided by the area of the circular foot constitutes 0.3 psi of downward pressure from the circular foot on the granite base. Specimens at least as large as the circular foot are analyzed between the circular foot and granite base.

## Z-Compliance Index and Percent Recovery Measurement Method

*Principle:* This method measures the ability of a web such as a nonwoven to be compressed in z-direction under applied pressure and then to recover to its original caliper after removing said applied pressure.

*Setup:* A vertically oriented electronic caliper tester having a precision of at least 0.01 mm with a 40 mm diameter circular foot may be used. The pressure exerted by the foot on the specimen is adjustable via the addition of pre-selected weights. Measurements are made at 0.85 $\pm$ 0.05 kPa and 15.4 $\pm$ 0.1kPa.

*Procedure:* Measurements are performed at 23°C $\pm$ 2°C and 50% $\pm$ 2% RH. All samples should be kept at least 24 hours in these conditions to equilibrate before conducting the tests, unless indicated otherwise. If possible, measurements are made on the sample specimen before it is integrated in an absorbent article. If this is not possible, care should be exerted when excising the sample to not impart any contamination or distortion to the test sample layer during the removal the material from other layers (using cryogenic spray, such as Cyto-Freeze, Control Company, Houston, Texas, if needed). The sample specimen is cut from to a square sample with a width of about 80 mm (or alternatively in case the material is not available in the suitable size in a material specimen with a width of about 50 mm).

The square sample specimen is positioned centered under the caliper foot and the caliper at 0.85 $\pm$ 0.05 kPa (P1) is measured and recorded to the nearest 0.01 mm (C1). Without removing the sample from the equipment, the pressure is increased to 15.4 $\pm$ 0.1 kPa (P2) and the caliper measured and recorded to the nearest 0.01 mm (C2). The pressure may be increased by adding a suitable weight on the caliper foot. Again without moving the sample, the exerted pressure is reduced back to 0.85 $\pm$ 0.05 kPa (for example by removing the extra weight) and the caliper measured a third time (C3) and recorded to the nearest 0.01mm.

For the specimen being measured, the compliance index is defined as:

$$\text{Z-compliance index} = (C1\text{-}C2) \, / \, (P2\text{-}P1)$$

and is recorded to the nearest 0.1 mm$^3$/N.

The recovery is calculated as:

$$\text{recovery} = C3 \, / \, C1 * 100\%$$

expressed in percent and recorded to the nearest 0.1%.

The procedure above is conducted on five like specimens of the same nonwoven. The arithmetic mean of the compliance index values among the five specimens is calculated and reported to the nearest 0.1 mm$^3$/N as the Compliance Index. The arithmetic mean of percent recovery values among the five specimens is calculated and reported to the nearest 0.1 % as the Percent Recovery.

## In-Bag Stack Height Test

The In-Bag Stack Height of a package of absorbent articles is determined as follows:

**Equipment:** A thickness tester with a flat, rigid horizontal sliding plate is used. The thickness tester is configured so that the horizontal sliding plate moves freely in a vertical direction with the horizontal sliding plate always maintained in a horizontal orientation directly above a flat, rigid horizontal base plate. The thickness tester includes a suitable device for measuring the gap between the horizontal sliding plate and the horizontal base plate to within $\pm$ 0.5 mm. The horizontal sliding plate and the horizontal base plate are larger than the surface of the absorbent article package that contacts each plate, i.e. each plate extends past the contact surface of the absorbent article package in all directions. The horizontal sliding plate exerts a downward force of 850 $\pm$ 1 gram-force (8.34 N) on the absorbent article package, which may be achieved by placing a suitable weight on the center of the non-package-contacting top surface of the horizontal sliding plate so that the total mass of the sliding plate plus added weight is 850 $\pm$ 1grams. Such a testing apparatus is for example illustrated on Fig. 19 of US2008/0312624A1.

**Test Procedure:** Absorbent article packages are equilibrated at 21 $\pm$ 2 °C and 50 $\pm$ 5% relative humidity prior to measurement. The horizontal sliding plate is raised and an absorbent article package is placed centrally under the horizontal sliding plate in such a way that the absorbent articles within the package are in a horizontal orientation. Any handle or other packaging feature on the surfaces of the package that would contact either of the plates is folded flat against the surface of the package so as to minimize their impact on the measurement. The horizontal sliding plate is lowered slowly until it contacts the top surface of the package and then released. The gap between the horizontal plates is measured to within $\pm$ 0.5 mm ten seconds after releasing the horizontal sliding plate. Five identical packages (same size packages and same absorbent articles counts) are measured and the arithmetic mean is reported as the package width. The "In-Bag Stack Height" = (package width/absorbent article count per stack) $\times$ 10 is calculated and reported to within $\pm$ 0.5 mm.

**Misc**

Unless indicated otherwise, the description and claims refer to the absorbent core and article before use (i.e. dry, and not loaded with a fluid) and conditioned at least 24 hours at 21 °C +/- 2 °C and 50 +/- 5% Relative Humidity (RH).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. A personal hygiene absorbent article (20) having a front side (10), a back side (12), a longitudinal axis (80) notionally extending in a longitudinal direction from the middle of the front side to the middle of the back side, the article having a length (L) measured along the longitudinal axis, the article notionally having a front region (62) having a length of one third of the length of the article, a back region (64) having a length of one third of the length of the article, and a crotch region (63) between the front region and back region having a length of the remaining third of the length the article;

    the absorbent article comprising a liquid permeable topsheet (24), a liquid impermeable backsheet (25), a pair of fastening tapes (42) disposed symmetrically on each side of the longitudinal axis in the back region, wherein the fastening tapes can be releasably fastened to a landing zone (44) in the front region of the article to form a closed article having a waist opening and two leg openings; and

    an absorbent core (28) between the topsheet and the backsheet, wherein the absorbent core comprises an absorbent material layer (60) between an upper substrate layer (16) and a lower substrate layer (16'); the absorbent layer comprising:

        i) at least one crotch channel (26, 26', 26", 26''') substantially free of absorbent material and predominantly disposed in the crotch region (63) of the article; wherein the upper substrate layer and the lower substrate layer are bonded to each other through the crotch channel by a crotch channel bond (27) having a crotch channel bond strength;

        ii) at least one front channel (56, 56') substantially free of absorbent material and predominantly disposed in the front region (62) of the article, the front channel being at least partially superposed with the landing zone (44); wherein the upper substrate layer and the lower substrate layer are either not bonded to each other through the front channel (56') or are bonded to each other through the front channel (56) by a front channel bond (57) having a front channel bond strength, wherein the front channel bond strength is weaker than the crotch channel bond strength.

2. An absorbent article (20) according to claim 1, wherein the crotch channel bond (27) comprises first bonding means and an additional bonding means; and the front channel bond (57) comprises the first bonding means but not the additional bonding means.

3. An absorbent article according to claim 2, wherein the first bonding means is an hotmelt adhesive, and the additional bonding means include at least one selected from pressure application between the upper substrate layer and lower substrate layer in the crotch channel to improve the bonding of these layers by the adhesive while the adhesive is in the open state, a supplementary adhesive, thermo bonding, mechanical bonding, ultrasonic bonding, or any combinations thereof.

4. An absorbent article according to claim 3, wherein the hotmelt adhesive of the first bonding means is an inner core glue present between the absorbent material layer (60) and the upper substrate layer (16) and/or between the absorbent material (60) and the lower substrate layer (16').

5. An absorbent article according to any of the preceding claims, wherein the front channel and the crotch channel do not extend to any of the edges of the absorbent material layer.

6. An absorbent article according to any of the preceding claims, wherein the length (L26) of at least one crotch channel is at least twice as long as the length of at least one front channel (L56), as measured projected on the longitudinal axis.

7. An absorbent article according to any of the preceding claims, wherein at least one crotch channel has a length which is at least 50% of the length of the absorbent core (L'), as measured projected on the longitudinal axis.

8. An absorbent article according to any of the preceding claims, wherein the front channel and the crotch channel are separated by absorbent material so that the closest distance (d) between the channels is of at least 5 mm, in particular from 5 mm to 20 mm.

9. An absorbent article according to any of the preceding claims, wherein the projections of the front channel and the crotch channel on an imaginary line parallel to the longitudinal axis are either overlapping or separated by a distance (l) which is less than 10 mm, in particular between 0 mm and 8 mm.

10. An absorbent article according to any of the preceding claims, wherein the article further comprises an acquisition layer (52) between the topsheet (24) and the absorbent core (28), preferably wherein the acquisition layer is a nonwoven acquisition layer.

11. An absorbent article according to any of the preceding claims, wherein the absorbent article comprises a lower acquisition and distribution system ("lower ADS") between the absorbent material layer (60) and the backsheet (25), wherein the lower ADS either consists of the lower substrate layer of the core only, or alternatively wherein the lower ADS consists of the lower substrate layer and one or more additional layer, and wherein the basis weight of the lower ADS ranges from 20 g/m$^2$ to 120 g/m$^2$, preferably wherein the lower ADS comprises at least one lofty layer having a Z-Compliance Index greater than 4 and a Percentage Recovery greater than 50%, as measured by the Z-Compliance Index and Percent Recovery Measurement Method as described herein.

12. An absorbent article according to any of the preceding claims, wherein the crotch channel bond (27) has a Static Peel Force Time which is at least 20 minutes greater than the Static Peel Force Time of the front channel bond (57).

13. An absorbent article according to any of the preceding claims, comprising a pair of crotch channels symmetrically disposed relative to the longitudinal axis, wherein the crotch channels may be connected or disconnected to one another, and a pair of front channels symmetrically disposed relative to the longitudinal axis, wherein the front channels may be connected or disconnected to one another.

14. An absorbent article according to any of the preceding claims, comprising a first and a second crotch channels symmetrically disposed relative to the longitudinal axis and defining a central absorbent zone comprising absorbent material disposed between the first and the second crotch channels; and a first lateral absorbent zone and a second lateral absorbent zone comprising absorbent material and disposed laterally outwardly of the first crotch channel and the second crotch channel respectively, wherein the basis weight of the absorbent material in the central absorbent zone is higher than the basis weight of the absorbent material in each of the lateral absorbent zones for at least a first transversal section of the core having a first length in the longitudinal direction of at least 10 mm; and the basis weight of the absorbent material in the central absorbent zone is lower than the basis weight of the absorbent material in each of the lateral absorbent zones for at least a second transversal section of the core having a second length in the longitudinal direction of at least 10 mm.

15. A process for making an absorbent article according to any of the preceding claims 1-14, the process comprising the steps of:

i) providing an upper substrate layer and a lower substrate layer;
ii) optionally applying an hotmelt adhesive on the upper substrate layer and / or the lower substrate layer;
iii) depositing an absorbent material on at least one of the upper substrate layer or lower substrate layer to form an absorbent material layer, wherein the absorbent material layer comprises at least one crotch channel and a front channel, the channels being substantially free of absorbent material, and if an adhesive has been applied, this adhesive is present between the absorbent material layer and the upper substrate layer and / or lower substrate layer;
iv) bonding the upper substrate layer and the lower substrate layer to each other through the crotch channel by a crotch channel bond and optionally through the front channel by a weaker front channel bond,
v) forming one or more core wrap seals (280', 282', 284', 286') to form an absorbent core;
vi) assembling the absorbent core thus obtained with other absorbent article components including the topsheet, backsheet, the pair of fastening tapes and the landing zone so that the front channel is at least partially superposed with the landing zone to obtain an article according to any of the preceding claims 1-14.

**Patentansprüche**

1. Körperpflegeabsorptionsartikel (20) mit einer vorderseitigen Seite (10), einer rückseitigen Seite (12), einer Längsachse (80), die sich gedanklich in einer Längsrichtung von der Mitte der vorderseitigen Seite zu der Mitte der rückseitigen Seite erstreckt, wobei der Artikel eine Länge (L) aufweist, die entlang der Längsachse gemessen wird, wobei der Artikel gedanklich einen vorderseitigen Bereich (62) mit einer Länge von einem Drittel der Länge des Artikels, einen rückseitigen Bereich (64) mit einer Länge von einem Drittel der Länge des Artikels und einen Schrittbereich (63) zwischen dem vorderseitigen Bereich und dem rückseitigen Bereich mit einer Länge des verbleibenden Drittels der Länge des Artikels aufweist;

wobei der Absorptionsartikel eine flüssigkeitsdurchlässige Oberschicht (24), eine flüssigkeitsundurchlässige Unterschicht (25), ein Paar Befestigungsbänder (42), die symmetrisch auf jeder Seite der Längsachse in dem rückseitigen Bereich angeordnet sind, umfasst, wobei die Befestigungsbänder lösbar an einer Auftreffzone (44) in dem vorderseitigen Bereich des Artikels befestigt werden können, um einen geschlossenen Artikel mit einer Taillenöffnung und zwei Schenkel-öffnungen zu bilden; und einen Absorptionskern (28) zwischen der Oberschicht und der Unterschicht, wobei der Absorptionskern eine Absorptionsmaterialschicht (60) zwischen einer oberen Substratschicht (16) und einer unteren Substratschicht (16') umfasst; wobei die Absorptionsschicht umfasst:

i) mindestens einen Schrittkanal (26, 26', 26", 26‴), der im Wesentlichen frei von Absorptionsmaterial ist und überwiegend in dem Schrittbereich (63) des Artikels angeordnet ist; wobei die obere Substratschicht und die untere Substratschicht miteinander durch den Schrittkanal durch eine Schrittkanalbindung (27) mit einer Schrittkanalbindungsstärke gebunden sind;
ii) mindestens einen vorderseitigen Kanal (56, 56'), der im Wesentlichen frei von Absorptionsmaterial ist und überwiegend in dem vorderseitigen Bereich (62) des Artikels angeordnet ist, wobei der vorderseitige Kanal mindestens teilweise über der Auftreffzone (44) angeordnet ist; wobei die obere Substratschicht und die untere Substratschicht entweder nicht durch den vorderseitigen Kanal (56') aneinander gebunden sind oder durch den vorderseitigen Kanal (56) durch eine vorderseitige Kanalbindung (57) mit einer vorderseitigen Kanalbindungsstärke gebunden sind, wobei die vorderseitige Kanalbindungsstärke schwächer als die Schrittkanalbindungsstärke ist.

2. Absorptionsartikel (20) nach Anspruch 1, wobei die Schrittkanalbindung (27) ein erstes Bindungsmittel und ein zusätzliches Bindungsmittel umfasst; und die vorderseitige Kanalbindung (57) das erste Bindungsmittel, jedoch nicht das zusätzliche Bindungsmittel umfasst.

3. Absorptionsartikel nach Anspruch 2, wobei das erste Bindungsmittel ein Hotmelt-Klebstoff ist und das zusätzliche Bindungsmittel mindestens eines einschließt, das aus einer Druckanwendung zwischen der oberen Substratschicht und der unteren Substratschicht in dem Schrittkanal, um die Bindung dieser Schichten durch den Klebstoff zu verbessern, während sich der Klebstoff in dem offenen Zustand befindet, einem zusätzlichen Klebstoff, einer Thermobindung, einer mechanische Bindung, einer Ultraschallbindung oder einer beliebigen Kombinationen davon ausgewählt ist.

4. Absorptionsartikel nach Anspruch 3, wobei der Hotmelt-Klebstoff des ersten Bindungsmittels ein Innenkernklebstoff ist, der zwischen der Absorptionsmaterialschicht (60) und der oberen Substratschicht (16) und/oder zwischen dem Absorptionsmaterial (60) und der unteren Substratschicht (16') vorliegt.

5. Absorptionsartikel nach einem der vorstehenden Ansprüche, wobei sich der vorderseitige Kanal und der Schrittkanal zu keiner der Kanten der Absorptionsmaterialschicht erstrecken.

6. Absorptionsartikel nach einem der vorstehenden Ansprüche, wobei die Länge (L26) von mindestens einem Schrittkanal mindestens doppelt so lang wie die Länge von mindestens einem vorderseitigen Kanal (L56) ist, was auf die Längsachse projiziert gemessen wird.

7. Absorptionsartikel nach einem der vorstehenden Ansprüche, wobei mindestens ein Schrittkanal eine Länge aufweist, die mindestens 50 % der Länge des Absorptionskerns (L') beträgt, was auf die Längsachse projiziert gemessen wird.

8. Absorptionsartikel nach einem der vorstehenden Ansprüche, wobei der vorderseitige Kanal und der Schrittkanal durch Absorptionsmaterial getrennt sind, sodass der nächstliegende Abstand (d) zwischen den Kanälen mindestens 5 mm, insbesondere 5 mm bis 20 mm, beträgt.

9. Absorptionsartikel nach einem der vorstehenden Ansprüche, wobei die Projektionen des vorderseitigen Kanals und des Schrittkanals auf einer gedachten Linie parallel zu der Längsachse entweder überlappen oder durch einen Abstand (I) getrennt sind, der weniger als 10 mm, insbesondere zwischen 0 mm und 8 mm, beträgt.

10. Absorptionsartikel nach einem der vorstehenden Ansprüche, wobei der Artikel ferner eine Aufnahmeschicht (52) zwischen der Oberschicht (24) und dem Absorptionskern (28) umfasst, wobei die Aufnahmeschicht vorzugsweise eine Vliesaufnahmeschicht ist.

**11.** Absorptionsartikel nach einem der vorstehenden Ansprüche, wobei der Absorptionsartikel ein unteres Aufnahme- und Verteilungssystem ("unteres ADS") zwischen der Absorptionsmaterialschicht (60) und der Unterschicht (25) umfasst, wobei das untere ADS entweder nur aus der unteren Substratschicht des Kerns besteht oder wobei das untere ADS alternativ aus der unteren Substratschicht und einer oder mehreren zusätzlichen Schichten besteht, und wobei das Basisgewicht des unteren ADS im Bereich von 20 g/m$^2$ liegt bis 120 g/m$^2$ liegt, wobei das untere ADS vorzugsweise mindestens eine untere Schicht mit einem Z-Compliance-Index von mehr als 4 und einer prozentualen Rückformung von mehr als 50 % umfasst, was durch das Messverfahren des Z-Compliance-Index und der prozentualen Rückformung, wie hierin beschrieben, gemessen wird.

**12.** Absorptionsartikel nach einem der vorstehenden Ansprüche, wobei die Schrittkanalbindung (27) eine Statikschälkraftzeit aufweist, die mindestens 20 Minuten mehr als die Statikschälkraftzeit der vorderseitigen Kanalbindung (57), die durch das hierin beschriebene Statikschälkraftzeitmessverfahren gemessen wird, beträgt.

**13.** Absorptionsartikel nach einem der vorstehenden Ansprüche, umfassend ein Paar Schrittkanäle, die symmetrisch bezüglich der Längsachse angeordnet sind, wobei die Schrittkanäle miteinander verbunden oder voneinander getrennt sein können, und ein Paar von vorderseitigen Kanälen, die symmetrisch bezüglich der Längsachse angeordnet sind, wobei die vorderseitigen Kanäle miteinander verbunden oder voneinander getrennt sein können.

**14.** Absorptionsartikel nach einem der vorstehenden Ansprüche, umfassend einen ersten und einen zweiten Schrittkanal, die symmetrisch bezüglich der Längsachse angeordnet sind und eine zentrale Absorptionszone definieren, die Absorptionsmaterial umfasst, das zwischen dem ersten und dem zweiten Schrittkanal angeordnet ist; und eine erste seitliche Absorptionszone und eine zweite seitliche Absorptionszone, die Absorptionsmaterial umfassen und seitlich außerhalb des ersten Schrittkanals und des zweiten Schrittkanals angeordnet sind, wobei das Basisgewicht des Absorptionsmaterials in der zentralen Absorptionszone höher als das Basisgewicht des Absorptionsmaterials in jeder der seitlichen Absorptionszonen für mindestens einen ersten Querschnitt des Kerns mit einer ersten Länge in der Längsrichtung von mindestens 10 mm ist; und das Basisgewicht des Absorptionsmaterials in der zentralen Absorptionszone niedriger als das Basisgewicht des Absorptionsmaterials in jeder der seitlichen Absorptionszonen für mindestens einen zweiten Querschnitt des Kerns mit einer zweiten Länge in der Längsrichtung von mindestens 10 mm ist.

**15.** Verfahren zum Herstellen eines Absorptionsartikels nach einem der vorstehenden Ansprüche 1 bis 14, wobei das Verfahren die Schritte umfasst:

i) Bereitstellen einer oberen Substratschicht und einer unteren Substratschicht;
ii) wahlweises Aufbringen eines Hotmelt-Klebstoffs auf die obere Substratschicht und/oder die untere Substratschicht;
iii) Abscheiden eines Absorptionsmaterials auf mindestens eine der oberen Substratschicht oder der unteren Substratschicht, um eine Absorptionsmaterialschicht zu bilden, wobei die Absorptionsmaterialschicht mindestens einen Schrittkanal und einen vorderseitigen Kanal umfasst, wobei die Kanäle im Wesentlichen frei von Absorptionsmaterial sind, und, wenn ein Klebstoff aufgebracht worden ist, dieser Klebstoff zwischen der Absorptionsmaterialschicht und der oberen Substratschicht und/oder der unteren Substratschicht vorliegt;
iv) Aneinanderbinden der oberen Substratschicht und der unteren Substratschicht durch den Schrittkanal durch eine Schrittkanalbindung und wahlweise durch den vorderseitigen Kanal durch eine schwächere vorderseitige Kanalbindung,
v) Bilden einer oder mehrerer Kernumwicklungsdichtungen (280', 282', 284', 286'), um einen Absorptionskern zu bilden;
vi) Zusammenbauen des somit erhaltenen Absorptionskerns mit anderen Absorptionsartikelbestandteilen, einschließlich der Oberschicht, der Unterschicht, des Paars Befestigungsbänder und der Auftreffzone, sodass der vorderseitige Kanal mindestens teilweise über der Auftreffzone angeordnet ist, um einen Artikel nach einem der vorstehenden Ansprüche 1 bis 14 zu erhalten.

## Revendications

**1.** Article d'hygiène personnelle absorbant (20) ayant un côté avant (10), un côté arrière (12), un axe longitudinal (80) s'étendant théoriquement dans une direction longitudinale du milieu du côté avant au milieu du côté arrière, l'article ayant une longueur (L) mesurée le long de l'axe longitudinal, l'article ayant théoriquement une région antérieure (62) ayant une longueur d'un tiers de la longueur de l'article, une région postérieure (64) ayant une longueur d'un tiers de la longueur de l'article, et une région d'entrejambe (63) entre la région antérieure et la région postérieure ayant une longueur du tiers restant de la longueur de l'article ;

l'article absorbant comprenant une feuille de dessus perméable aux liquides (24), une feuille de fond imperméable aux liquides (25), une paire de rubans de fixation (42) disposés symétriquement de chaque côté de l'axe longitudinal dans la région postérieure, dans lequel les rubans de fixation peuvent être fixés de manière amovible à une zone de réception (44) dans la région antérieure de l'article pour former un article fermé ayant une ouverture de ceinture et deux ouvertures de jambe ; et

une âme absorbante (28) entre la feuille de dessus et la feuille de fond, dans lequel l'âme absorbante comprend une couche de matériau absorbant (60) entre une couche de substrat supérieure (16) et une couche de substrat inférieure (16') ; la couche absorbante comprenant :

    i) au moins un canal d'entrejambe (26, 26', 26", 26‴) essentiellement dépourvu de matériau absorbant et disposé principalement dans la région d'entrejambe (63) de l'article ; dans lequel la couche de substrat supérieure et la couche de substrat inférieure sont liées l'une à l'autre à travers le canal d'entrejambe par une liaison de canal d'entrejambe (27) ayant une force de liaison de canal d'entrejambe ;

    ii) au moins un canal avant (56, 56') essentiellement dépourvu de matériau absorbant et disposé principalement dans la région antérieure (62) de l'article, le canal avant étant au moins partiellement superposé avec la zone de réception (44) ; dans lequel la couche de substrat supérieure et la couche de substrat inférieure ne sont pas liées l'une à l'autre à travers le canal avant (56') ou sont liées l'une à l'autre à travers le canal avant (56) par une liaison de canal avant (57) ayant une force de liaison de canal avant, dans lequel la force de liaison de canal avant est plus faible que la force de liaison de canal d'entrejambe.

2. Article absorbant (20) selon la revendication 1, dans lequel la liaison de canal d'entrejambe (27) comprend un premier moyen de liaison et un moyen de liaison supplémentaire ; et la liaison de canal avant (57) comprend le premier moyen de liaison mais pas le moyen de liaison supplémentaire.

3. Article absorbant selon la revendication 2, dans lequel le premier moyen de liaison est un adhésif thermofusible, et le moyen de liaison supplémentaire en inclut au moins un choisi parmi une application de pression entre la couche de substrat supérieure et la couche de substrat inférieure dans le canal d'entrejambe pour améliorer la liaison de ces couches par l'adhésif alors que l'adhésif est à l'état ouvert, un adhésif supplémentaire, un thermoliage, une liaison mécanique, une liaison par ultrasons, ou de quelconques combinaisons de ceux-ci.

4. Article absorbant selon la revendication 3, dans lequel l'adhésif thermofusible du premier moyen de liaison est une colle d'âme interne présente entre la couche de matériau absorbant (60) et la couche de substrat supérieure (16) et/ou entre le matériau absorbant (60) et la couche de substrat inférieure (16').

5. Article absorbant selon l'une quelconque des revendications précédentes, dans lequel le canal avant et le canal d'entrejambe ne s'étendent pas jusqu'à l'un quelconque des bords de la couche de matériau absorbant.

6. Article absorbant selon l'une quelconque des revendications précédentes, dans lequel la longueur (L26) d'au moins un canal d'entrejambe est au moins deux fois plus longue que la longueur d'au moins un canal avant (L56), telles que mesurées en projection sur l'axe longitudinal.

7. Article absorbant selon l'une quelconque des revendications précédentes, dans lequel au moins un canal d'entrejambe a une longueur qui est d'au moins 50 % de la longueur de l'âme absorbante (L'), telles que mesurées en projection sur l'axe longitudinal.

8. Article absorbant selon l'une quelconque des revendications précédentes, dans lequel le canal avant et le canal d'entrejambe sont séparés par du matériau absorbant de sorte que la distance (d) la plus proche entre les canaux est d'au moins 5 mm, en particulier de 5 mm à 20 mm.

9. Article absorbant selon l'une quelconque des revendications précédentes, dans lequel les projections du canal avant et du canal d'entrejambe sur une ligne imaginaire parallèle à l'axe longitudinal se chevauchent ou sont séparées d'une distance (l) qui est de moins de 10 mm, en particulier comprise entre 0 mm et 8 mm.

10. Article absorbant selon l'une quelconque des revendications précédentes, dans lequel l'article comprend en outre une couche d'acquisition (52) entre la feuille de dessus (24) et l'âme absorbante (28), de préférence dans lequel la couche d'acquisition est une couche d'acquisition non tissée.

11. Article absorbant selon l'une quelconque des revendications précédentes, dans lequel l'article absorbant comprend un système d'acquisition et de distribution inférieur (« ADS inférieur ») entre la couche de matériau absorbant (60) et la feuille de fond (25), dans lequel l'ADS inférieur est constitué de la couche de substrat inférieure de l'âme uniquement, ou alternativement dans lequel l'ADS inférieur est constitué de la couche de substrat inférieure et d'une ou plusieurs couches supplémentaires, et dans lequel la masse surfacique de l'ADS inférieur va de 20 g/m$^2$ à 120 g/m$^2$, de préférence dans lequel l'ADS inférieur comprend au moins une couche élastique ayant un indice de conformance suivant Z supérieur à 4 et un pourcentage de recouvrance supérieur à 50 %, tels que mesurés par le procédé de mesure d'indice de conformance suivant Z et de pourcentage de recouvrance tel que décrit ici.

**12.** Article absorbant selon l'une quelconque des revendications précédentes, dans lequel la liaison de canal d'entrejambe (27) a un temps sous une force de pelage statique qui est supérieur d'au moins 20 minutes au temps sous une force de pelage statique de la liaison de canal avant (57), tels que mesurés par le procédé de mesure de temps sous une force de pelage statique décrit ici.

**13.** Article absorbant selon l'une quelconque des revendications précédentes, comprenant une paire de canaux d'entrejambe disposés symétriquement par rapport à l'axe longitudinal, dans lequel les canaux d'entrejambe peuvent être connectés l'un à l'autre ou déconnectés l'un de l'autre, et une paire de canaux avant disposés symétriquement par rapport à l'axe longitudinal, dans lequel les canaux avant peuvent être connectés l'un à autre ou déconnectés l'un de l'autre.

**14.** Article absorbant selon l'une quelconque des revendications précédentes, comprenant des premier et second canaux d'entrejambe disposés symétriquement par rapport à l'axe longitudinal et définissant une zone absorbante centrale comprenant du matériau absorbant disposé entre les premier et second canaux d'entrejambe ; et une première zone absorbante latérale et une seconde zone absorbante latérale comprenant du matériau absorbant et disposées latéralement vers l'extérieur du premier canal d'entrejambe et du second canal d'entrejambe respectivement, dans lequel la masse surfacique du matériau absorbant dans la zone absorbante centrale est plus élevée que la masse surfacique du matériau absorbant dans chacune des zones absorbantes latérales pour au moins une première section transversale de l'âme ayant une première longueur dans la direction longitudinale d'au moins 10 mm ; et la masse surfacique du matériau absorbant dans la zone absorbante centrale est inférieure à la masse surfacique du matériau absorbant dans chacune des zones absorbantes latérales pour au moins une seconde section transversale de l'âme ayant une seconde longueur dans la direction longitudinale d'au moins 10 mm.

**15.** Procédé de fabrication d'un article absorbant selon l'une quelconque des revendications 1 à 14 précédentes, le procédé comprenant les étapes consistant à :

i) fournir une couche de substrat supérieure et une couche de substrat inférieure ;

ii) éventuellement appliquer un adhésif thermofusible sur la couche de substrat supérieure et/ou la couche de substrat inférieure ;

iii) déposer un matériau absorbant sur au moins l'une de la couche de substrat supérieure ou de la couche de substrat inférieure pour former une couche de matériau absorbant, dans lequel la couche de matériau absorbant comprend au moins un canal d'entrejambe et un canal avant, les canaux étant essentiellement dépourvus de matériau absorbant, et si un adhésif a été appliqué, cet adhésif est présent entre la couche de matériau absorbant et la couche de substrat supérieure et/ou la couche de substrat inférieure ;

iv) lier la couche de substrat supérieure et la couche de substrat inférieure l'une à l'autre à travers le canal d'entrejambe par une liaison de canal d'entrejambe et éventuellement à travers le canal avant par une liaison de canal avant plus faible,

v) former un ou plusieurs joints d'enveloppe d'âme (280', 282', 284', 286') pour former une âme absorbante ;

vi) assembler l'âme absorbante ainsi obtenue avec d'autres composants d'article absorbant incluant la feuille de dessus, la feuille de fond, la paire de rubans de fixation et la zone de réception de sorte que le canal avant est au moins partiellement superposé avec la zone de réception pour obtenir un article selon l'une quelconque des revendications 1 à 14 précédentes.

# Fig. 1

**Fig. 2**

**Fig. 3**

**Fig. 4a**

**Fig. 4b**

## Fig. 5

**Fig. 6**

## Fig. 7

## Fig. 8

# Fig. 9

## Fig. 10

101    104

102    103

27

100

## Fig. 11

103    16

16    103'

102

16'    27    16'

EP 3 944 844 B1

Fig. 12

36

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5433715 A, Tanzer
- WO 2012052172 A, Van Malderen
- US 2008312617 A, Hundorf
- WO 2012170778 A1
- WO 2012170779 A1, Rosati
- WO 201493129 A, Roe
- WO 2014200794 A, Bianchi
- WO 2019083767 A1, Bianchi
- WO 2006138725 A2, Lam
- US 3848594 A
- US 4662875 A
- US 4846815 A
- US 4894060 A
- US 4946527 A
- US 5151092 A
- US 5221274 A, Buell
- US 6432098 B
- US 4963140 A, Robertson
- WO 2017189150 A1, Bianchi
- WO 2008155699 A, Hundorf
- WO 2004011723 A, Venturino
- WO 2012170798 A1, Jackels
- EP 2905000 A, Jackels
- EP 2905001 A, Armstrong-Ostle
- WO 9511652 A, Tanzer
- WO 2014093130 A1, Roe
- WO 2014093129 A, Roe
- US 7744576 B
- US 20110268932 A1
- US 20110319848 A1
- US 20110250413 A1
- EP 2886092 A, Stelzig
- US 6632504 B, Gillespie
- WO 2011163582 A, Rinnert
- US 3929135 A
- US 4324246 A
- US 4342314 A
- US 4463045 A
- US 5006394 A
- US 4609518 A
- US 4629643 A
- US 20140121623 A1
- US 20140121621 A1
- US 20140121624 A1
- US 20140121625 A1
- US 6645569 B
- US 6863933 B
- US 2003148684 A
- US 2005008839 A, Cramer
- US 7112621 B, Rohrbaugh
- US 5607760 A
- US 5609587 A
- US 5643588 A
- US 5968025 A
- US 6716441 B
- WO 9524173 A
- US 6075179 A
- WO 2014022362 A1
- WO 2014022652 A1
- US 5837352 A
- US 5843056 A
- WO 2014168810 A1, Bianchi
- US 3860003 A
- US 4808178 A, Aziz
- US 4909803 A, Aziz
- US 4695278 A, Lawson
- US 4795454 A, Dragoo
- US 7786341 B
- US 20030105190 A, Diehl
- WO 200071067 A, KIM DOO-HONG
- US 20080312622 A1, Hundorf
- US 5549791 A
- US 5137537 A
- WO 9534329 A
- US 2007118087 A
- WO 2019241009 A1, P&G, Tally
- WO 2014093310 A
- WO 2012177400 A
- WO 2012177401 A, Lawson
- US 4515595 A
- US 4710189 A
- US 6336922 B, VanGompel
- EP 2532329 A
- EP 2905000 A1